# EUROPEAN PATENT SPECIFICATION

(11) **EP 1 789 384 B1**
(45) Date of publication and mention of the grant of the patent: **15.10.2008**
(21) Application number: 05770141.9
(22) Date of filing: 22.08.2005
(51) Int. Cl.: C07C 401/00, C07J 9/00

(54) **EPIMERISATION OF ALLYLIC ALCOHOLS**
EPIMERISATION VON ALLYLALKOHOLEN
EPIMERISATION D'ALCOOLS ALLYLIQUES

(30) Priority: 01.09.2004 US 606135 P
(43) Date of publication of application: 30.05.2007
(73) Proprietor: LEO PHARMA A/S, 2750 Ballerup (DK)
(72) Inventor: PEDERSEN, Henrik, DK-4340 Tølløse (DK); BRETTING, Claus, Aage, Svensgaard, DK-2000 Frederiksberg (DK); BINDERUP, Ernst, Torndal, DK-2630 Taastrup (DK)
(86) International application number: PCT/DK2005/000533
(87) International publication number: WO 2006/024296

(56) References cited:
- WO-A-03/106412
- US-A- 4 866 048
- US-A- 5 763 426

## Description

### FIELD OF THE INVENTION

The present invention relates to a novel epimerisation method of compounds, for example compounds useful for the synthesis of vitamin D analogues having a hydroxyl substituent on an asymmetric allylic carbon, such as at the 24 position. The present invention relates further to the use of intermediates produced with said method for making calcipotriol {(5Z, 7E, 22E, 24S)-24-cyclopropyl-9,10-secochola-5,7,10(19),22-tetraene-1α-3β-24-triol} or calcipotriol monohydrate.

### BACKGROUND OF THE INVENTION

Calcipotriol or calcipotriene (structure I) [CAS 112965-21-6] shows a strong activity in inhibiting undesirable proliferation of epidermal keratinocytes [F.A.C.M. Castelijins, M.J. Gerritsen, I.M.J.J. van Vlijmen-Willems, P.J. van Erp, P.C.M. van de Kerkhof; Acta Derm. Venereol. 79, 11, 1999]. The efficiency of calcipotriol and calcipotriol monohydrate (I-hydrate) in the treatment of psoriasis was shown in a number of clinical trials [D.M. Ashcroft et al.; Brit. Med. J. 320, 963-67, 2000] and calcipotriol is currently used in several commercial drug formulations.

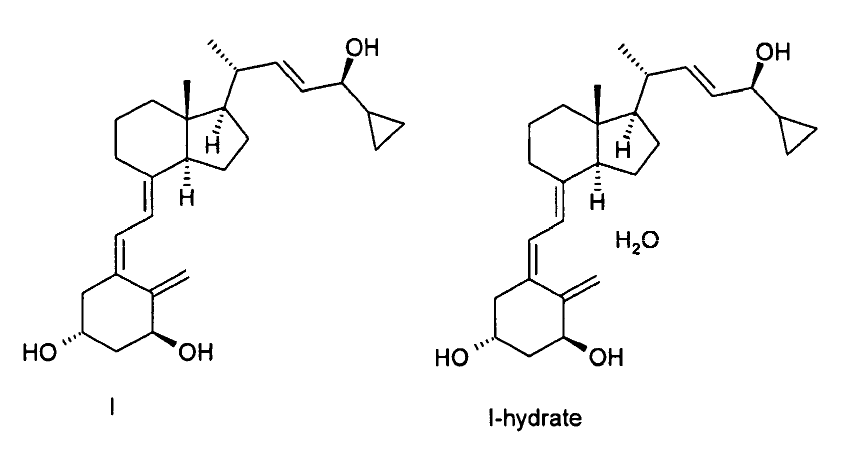

In the preparation of calcipotriol, the specific stereochemistry for the hydroxyl group at C-24 is necessary for full expression of the biological activity.

In the previously disclosed process for making calcipotriol I, the hydroxyl protected C-24 ketone of I is reduced to give a mixture of the C-24 epimers IIIa and IIIb, wherein R₁ and R₂ may be the same or different and represent hydrogen or a hydroxy protecting group, as outlined in WO 87/00834 & M.J. Calverley; Tetrahedron, 43 (20), 4609-19, 1987).

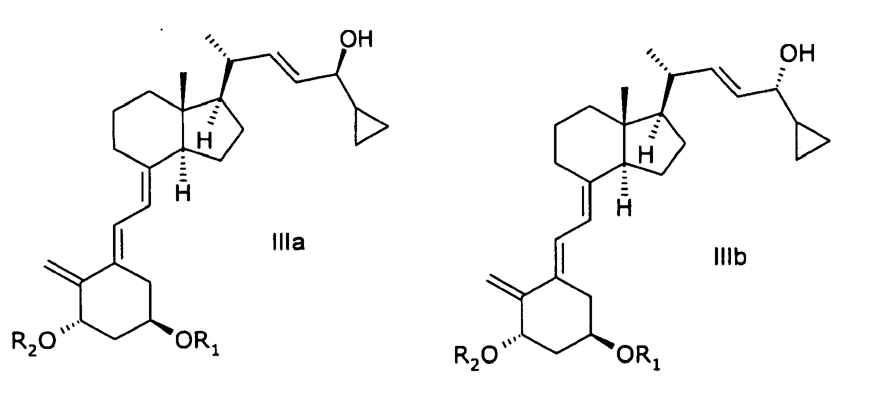

The desired epimer IIIa is then separated, for example by chromatography, followed by photoisomerisation and removal of the protecting groups to yield calcipotriol, whereas the undesired epimer IIIb is discarded. Thus, waste is high and the overall yield and process productivity are correspondingly low.
WO 03/106412 describes a method whereby the undesired epimer IIIb can be converted into an C-24 ester of IIIb, which then can be epimerised by contacting said ester with an epimerisation active-solid. After hydrolysis of the mixture of C-24 esters of IIIa and IIIb, a mixture enriched in the desired epimer IIIa can be obtained and the enriched mixture can be recycled to the separation step. This epimerisation process has the disadvantage that it involves two additional chemical transformations, namely the esterification and the saponification of IIIb, which makes this process economically unfavourable, especially on an industrial scale.
WO 94/07853 discloses a number of vitamin D analogues having a hydroxyl substituent on an asymmetric allylic carbon at the 24 position, but no method for epimerising said alcohols. A specific C-24 epimer described in WO 94/07853 (Tisocalcitate) is currently in Phase 2 clinical trials.

### SUMMARY OF THE INVENTION

The present invention surprisingly provides a novel process to epimerise alcohols of compounds having a hydroxyl substituent on an asymmetric allylic carbon, such as compounds useful for the synthesis of vitamin D analogues where the hydroxyl substituent is at the 24 position, in the presence of an acid and water. The vitamin D epimers obtainable by the method may be useful e.g. as intermediates in the synthesis of calcipotriol. The novel process employs inexpensive chemicals and is simple to operate on an industrial scale. Since the novel method allows the recycling of undesired C-24 hydroxyl epimers of calcipotriol precursors or derivatives, but avoids the additional esterification and saponification steps described in WO 03/106412, the overall yield and the process productivity may be improved.

In one aspect, this invention relates to a method of epimerising an epimer or epimeric mixture of a compound comprising a side chain of general structure A and/or B, 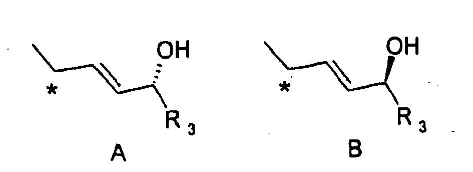 at the position of the carbon atom to which the hydroxy group and R₃ are attached to; by contacting said epimer or epimeric mixture with an acid in the presence of water;
wherein R₃ is (C₁-C₁₂)alkyl, (C₂-C₁₀)alkenyl, (C₂-C₂₀)alkynyl, or (C₃-C₂₀)cycloalkyl, optionally substituted with one or more substituents selected from the group consisting of (C₁-C₁₂)alkyl, -C(O)-O-(C₁-C₁₂)alkyl, halogen, hydroxy, methoxy, and ethoxy;
wherein the carbon marked with an asterisk is connected by a single bond to the C-17 carbon atom of a vitamin D analogue fragment;
or wherein the carbon marked with an asterisk is connected by a single bond to a fragment of a precursor for the synthesis of a vitamin D analogue at its C-17 analogous position;
wherein the fragment of a precursor for the synthesis of a vitamin D analogue is a fragment of a steroid ring system or a fragment of a CD ring system of a steroid;
wherein the fragment of the steroid ring system is represented by structure Q or R 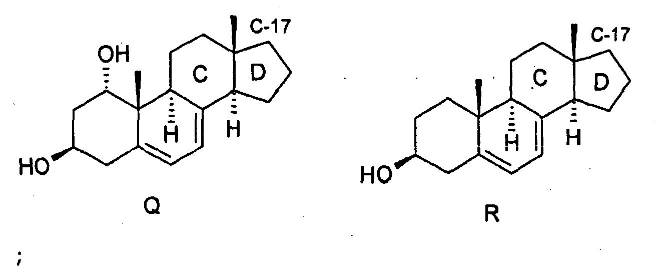 wherein the fragment of a CD ring system of a steroid is represented by structure E or P 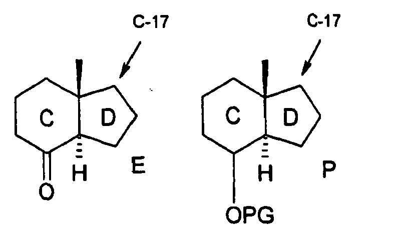 ; wherein PG represents hydrogen or a hydroxy protecting group;
wherein the vitamin D analogue fragment is represented by any one of the fragments F, G, H, J, K, or L 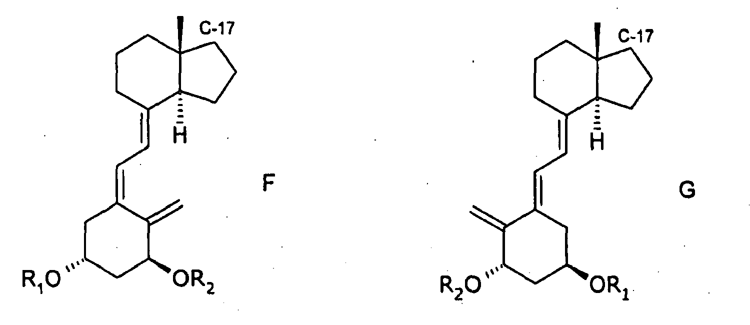 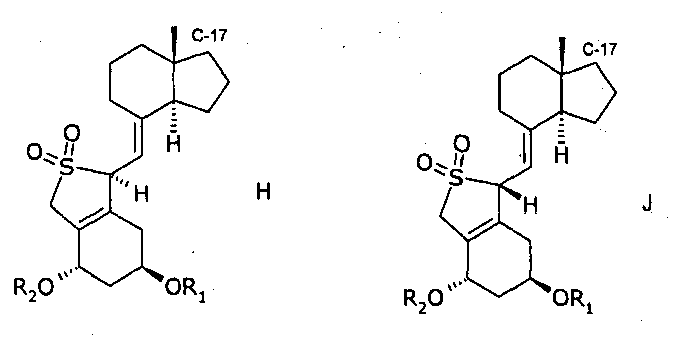 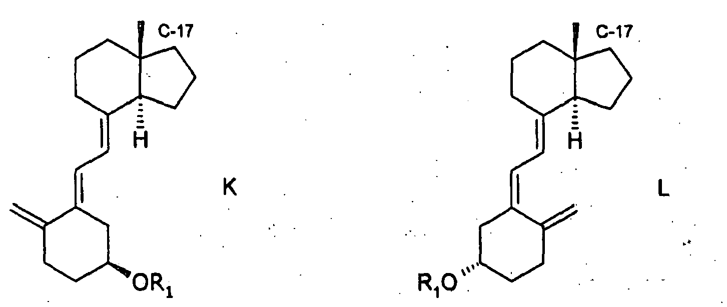 ; wherein R₁ and/or R₂ may be the same or different and represent hydrogen or a hydroxy protecting group.

In another aspect, this invention relates to a method of preparing calcipotriol or calcipotriol monohydrate comprising, in one or more steps, the method above.

In yet another aspect, this invention relates to a method for producing calcipotriol or calcipotriol monohydrate comprising the steps of:
(i) epimerising a vitamin D-analogue of general structure IIb, 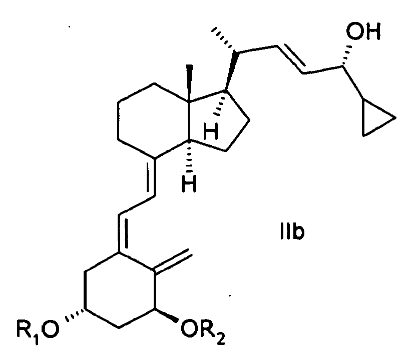 wherein R₁ and R₂ may be the same or different and represent hydrogen or a hydroxy protecting group,
   with an acid in the presence of water to give a mixture of compounds of general structure IIa and IIb, wherein R₁ and R₂ are as defined above; 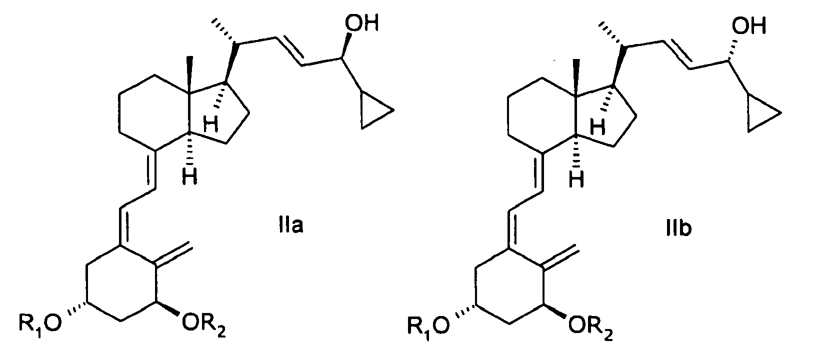
(ii) optionally separating the compound of general structure IIa from the mixture of compounds of general structure IIa and IIb, wherein R₁ and R₂ are as defined above;
(iii) when R₁ and/or R₂ are not hydrogen, removing the hydroxy protecting group(s) R₁ and/or R₂ of the compound of general structure IIa to generate calcipotriol ; and
(iv) optionally crystallising the calcipotriol from a mixture of an organic solvent and water to give calcipotriol monohydrate.

In yet another aspect, this invention relates to a method for producing calcipotriol or calcipotriol monohydrate comprising the steps of:
(i) epimerising a vitamin D-analogue of general structure IIIb, 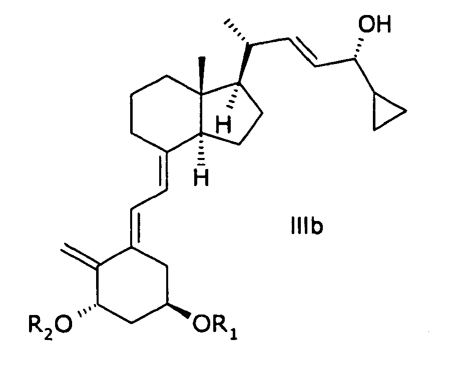 wherein R₁ and R₂ may be the same or different and represent hydrogen or a hydroxy protecting group,
   with an acid in the presence of water to give a mixture of compounds of general structure IIIa and IIIb,
   wherein R₁ and R₂ are as defined above; 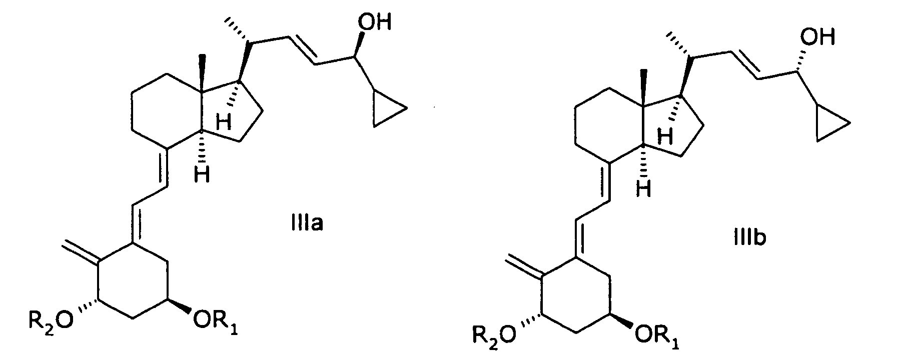
(ii) optionally separating the compound of general structure IIIa from the mixture of compounds of general structure IIIa and IIIb;
(iii) photoisomerising the compound of general structure IIIa to the compound of general structure IIa, 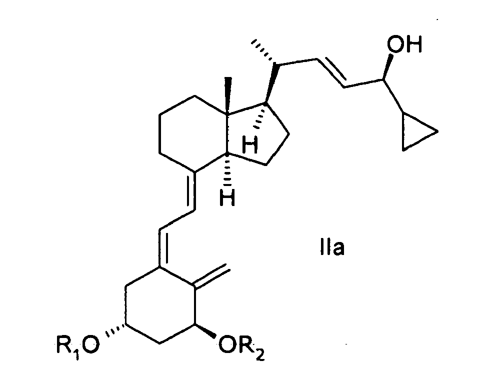 wherein R₁ and R₂ are as defined above;
(iv) when R₁ and/or R₂ are not hydrogen, removing the hydroxy protecting group(s) R₁ and/or R₂ of the compound of general structure IIa to generate calcipotriol; and
(v) optionally crystallising the calcipotriol from a mixture of an organic solvent and water to give calcipotriol monohydrate;
wherein steps (iii) and (iv) may be carried out in reverse order.

In yet another aspect, this invention relates to a method for producing calcipotriol or calcipotriol monohydrate comprising the steps of:
(i) epimerising a vitamin D-analogue of general structure IVba and/or IVbb, 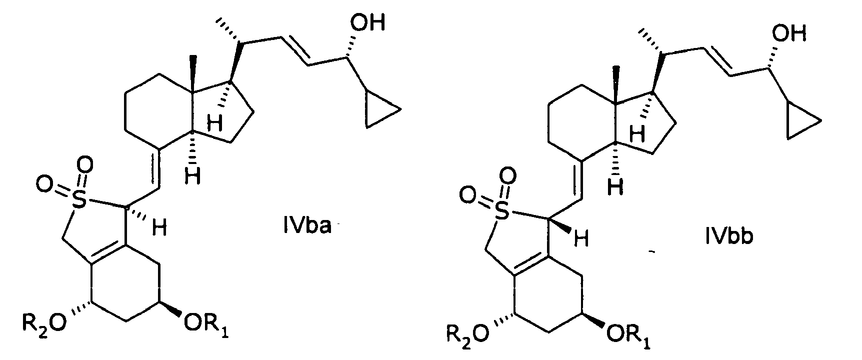 wherein R₁ and R₂ may be the same or different and represent hydrogen or a hydroxy protecting group,
   with an acid in the presence of water to give a mixture of compounds of general structure IVba and/or IVbb and IVaa and/or IVab,
   wherein R₁ and R₂ are as defined above; 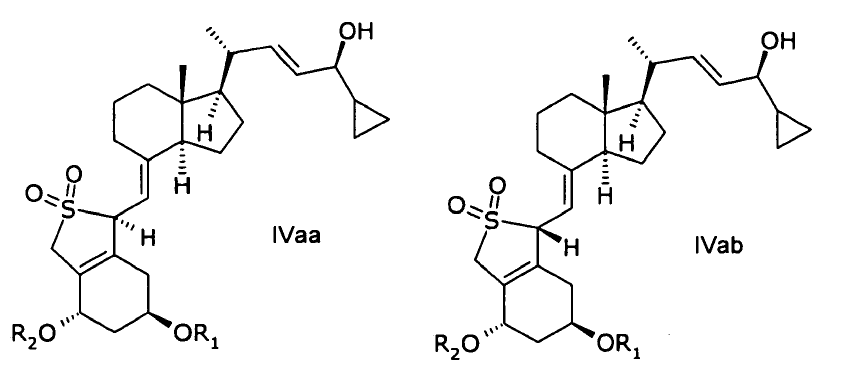
(ii) optionally separating the compounds of general structure IVaa and/or IVab from the reaction mixture;
(iii) heating the compounds of general structure IVaa and/or IVab above 60°C in the presence of a base to give a compound of general structure IIIa, 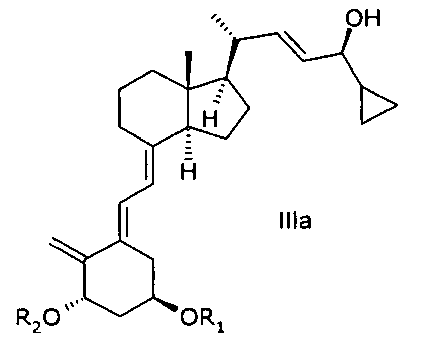 wherein R₁ and R₂ are as defined above;
(iv) photoisomerising the compound of general structure IIIa to the compound of general structure IIa, 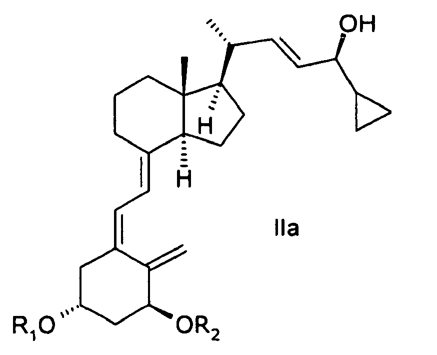 wherein R₁ and R₂ are as defined above;
(v) when R₁ and/or R₂ are not hydrogen, removing the hydroxy protecting group(s) R₁ and/or R₂ of the compound of general structure IIa to generate calcipotriol; and
(vi) optionally crystallising the calcipotriol from a mixture of an organic solvent and water to give calcipotriol monohydrate;
wherein steps (iv) and (v) may be carried out in reverse order.

In yet another aspect, this invention relates to the use of a method as above in the manufacture of calcipotriol or calcipotriol monohydrate.

In yet another aspect, this invention relates a method as above for the manufacture of pharmaceutical formulation or medicament containing calcipotriol or calcipotriol monohydrate, such as a cream, an ointment or a gel.

In yet another aspect, this invention relates to a method of preparing calcipotriol or calcipotriol monohydrate, the method comprising a method as above.

### DETAILED DESCRIPTION OF THE INVENTION

### Definitions

As used herein a "hydroxy protecting group" includes any group which forms a derivative that is stable to the projected reactions wherein said hydroxy protecting group can be selectively removed by reagents that do not attack the regenerated hydroxy group. Said derivative can be obtained by selective reaction of a hydroxy protecting agent with a hydroxy group. Silyl derivatives, such as *tert*-butyldimethylsilyl forming silyl ethers are examples of hydroxy protecting groups. Silyl chlorides such as *tert*-butyldimethylsilyl chloride (TBSCI), trimethylsilylchloride, triethylsilylchloride, diphenylmethylsilylchloride, triisopropylsilylchloride, and *tert*-butyldiphenylsilylchloride are examples of hydroxy protecting agents. Hydrogen fluoride, such as aqueous HF in acetonitrile, or tetra n-butylammonium fluoride are examples of reagents which can remove silyl groups. Other hydroxy protecting groups include ethers, such as tetrahydropyranyl (THP) ether, including alkoxyalkyl ethers (acetals), such as methoxymethyl (MOM) ether, or benzyl ether, or esters, such as chloroacetate ester, trimethylacetate, acetate or benzoate ester. Non-limiting examples of hydroxy protecting groups and methods of protection and removal, all included in the scope of this application, can for example be found in "Protective Groups in Organic Synthesis", 3rd ed., T. W. Greene & P. G. M. Wuts eds., John Wiley 1999 and in "Protecting Groups", 1st ed., P.J. Kocienski, G. Thieme 2000, all of which are hereby incorporated by reference.

In the present context, the term "alkyl" is intended to indicate the radical obtained when one hydrogen atom is removed from a hydrocarbon. Said alkyl comprises 1-20, preferably 1-12, such as 1-7, such as 1-4 carbon atoms. The term includes the subclasses normal alkyl (n-alkyl), secondary and tertiary alkyl, such as methyl, ethyl, n-propyl, isopropyl, n-butyl, isobutyl, *sec*.-butyl, *tert*.-butyl, pentyl, isopentyl, hexyl, isohexyl, and the *tert*-butyldimethyl group.

The term "alkoxycarbonyl" is intended to indicate a radical of the formula -C(O)-O-R', wherein R' is alkyl as indicated above, e.g. methoxycarbonyl, ethoxycarbonyl, n-propoxycarbonyl, isopropoxycarbonyl, etc.

The term "cycloalkyl" is intended to indicate a saturated cycloalkane radical comprising 3-20 carbon atoms, preferably 3-10 carbon atoms, in particular 3-8 carbon atoms, such as 3-6 carbon atoms, e.g. cyclopropyl, cyclobutyl, cyclopentyl or cyclohexyl.

The term "alkenyl" is intended to indicate a mono-, di-, tri-, tetra- or pentaunsaturated hydrocarbon radical comprising 2-10 carbon atoms, in particular 2-6 carbon atoms, such as 2-4 carbon atoms, e.g. ethenyl, propenyl, butenyl, pentenyl or hexenyl.

The term "alkynyl" is intended to indicate a hydrocarbon radical comprising 1-5 triple C-C bonds and 2-20 carbon atoms, the alkane chain typically comprising 2-10 carbon atoms, in particular 2-6 carbon atoms, such as 2-4 carbon atoms, e.g. ethynyl, propynyl, butynyl, pentynyl or hexynyl.

The term "halogen" is intended to indicate a substituent form the 7^{th} main group of the periodic table, preferably fluoro, chloro and bromo.

As used herein, "vitamin D-analogue" includes any derivative of vitamin D₂ or D₃, such as 1α,25-dihydroxyvitamin D₂ or 1α,25-dihydroxyvitamin D₃, including derivatives wherein one or more of the A, C, or D ring are modified or/and where the side chain attached to C-17 is different from natural vitamin D₂ or D₃. Examples of vitamin D-analogues can for example be found in ["Vitamin D", D. Feldman, Ed., Academic Press, San Diego, USA, 1997] and [G.-D. Zhu et al., Chem. Rev. 1995, 95, 1877-1952] and references cited therein, all of which are hereby incorporated by reference, and include calcipotriol and its C-24 epimer and Tisocalcitate [9,10-Secocholesta-5,7,10(19),22-tetraene-25-carboxylic acid, 1,3,24-trihydroxy-, 1-methylethyl ester, (1α,3β,5Z,7E,22E,24R)] and its C-24 epimer.

As used herein, "vitamin D-analogue fragment" means a C-17 radical of a vitamin D-analogue as defined above without the side chain usually attached at C-17.

As used herein, "a precursor for the synthesis of a vitamin D-analogue" means any molecule useful in the synthesis of a vitamin D derivative as defined above, such as a starting material or intermediate, wherein part of the precursor molecule or the whole molecule, optionally after further chemical transformations, becomes incorporated into the final vitamin D-analogue. Examples include, but are not limited to steroid ring systems, such as ergosterol, cholesterol, or 7-dehydrocholesterol, or derivatives of the CD-rings of steroids, such as Grundmann's ketone or derivatives of Grundmann's ketone. Examples of precursors for the synthesis of a vitamin D-analogue can be found in [G.-D. Zhu et al., Chem. Rev. 1995, 95, 1877-1952] and references cited therein, all of which are hereby incorporated by reference. Examples of specific derivatives of CD-rings of steroids, which are in particular useful are the ring structures M and N illustrated below, wherein PG is hydrogen or a hydrogen protecting group as defined above, such as benzyl or benzoyl.

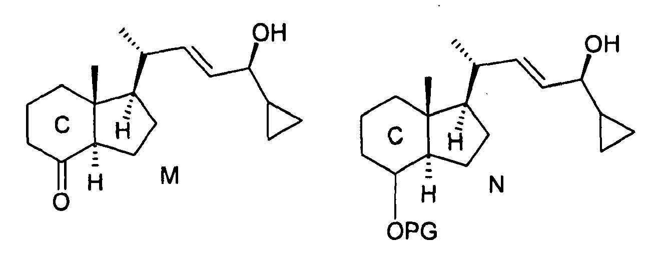

A C-17 analogous position of such a precursor is intended to mean the carbon atom of said precursor, which will correspond to the C-17 carbon atom in the final vitamin D-analogue.

As used herein, "a fragment of a precursor for the synthesis of a vitamin D-analogue" means a radical of a precursor for the synthesis of a vitamin D-analogue as defined above. For example a fragment of a precursor for the synthesis of a vitamin D-analogue may be a steroid ring system fragment.

Other examples of fragments of a precursor for the synthesis of a vitamin D-analogue are fragments of derivatives of the CD-rings of steroids.

As used herein, "separating a compound" includes the purification and/or isolation of a compound, e.g. to at least 90% purity, such as to at least 95% purity, such as 97% purity, 98% purity, or 99% purity. The term "separating a compound" also includes enhancing the concentration of the compound in a mixture of such compounds, optionally comprising solvents, such that the mixture is further enriched with a desired or preferred compound or isomer, such as an epimer, after said separation.

### Embodiments

In a preferred embodiment of the present invention R₁ and R₂ represent alkylsilyl or hydrogen.

In another preferred embodiment of the present invention R₁ and R₂ represent *tert-*butyldimethylsilyl.

In another preferred embodiment of the present invention R₃ is cyclopropyl or -C(CH₃)₂-C(O)-O-CH(CH₃)₂.

In another preferred embodiment of the present invention the configuration at the carbon atom C-20 of the side chain marked with an asterisk is (R) and the configuration at C-17 or the C-17 analogous position is the same as in natural vitamin D₃.

In another preferred embodiment of the present invention the epimer is compound IIIba or the epimeric mixture comprises compound IIIaa and IIIba.

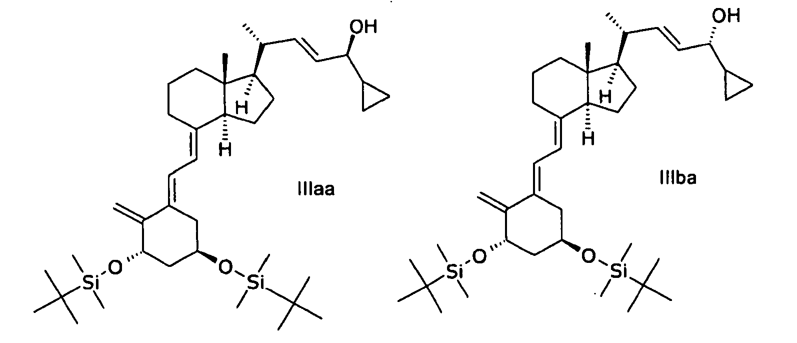

Further examples of specific molecules which can be epimerised at C-24 by contacting said molecules with an acid in the presence of water are compounds XX, XXI, XXII, XXIII, XXIV or their corresponding C-24 epimers, or mixtures of the C-24 epimers, wherein R₁ and R₂ may be the same or different and represent hydrogen or a hydroxy protecting group.

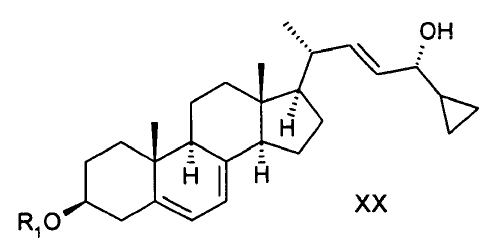

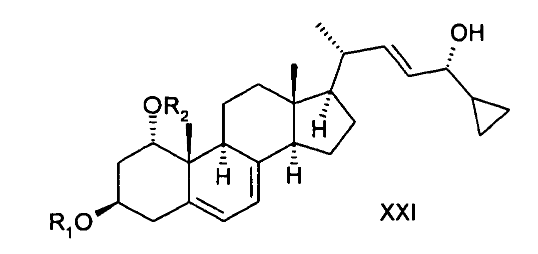

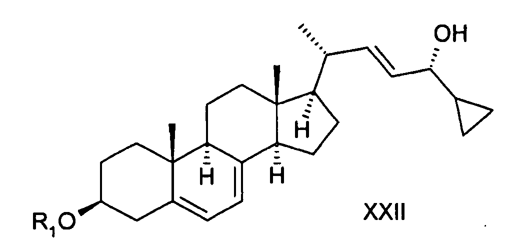

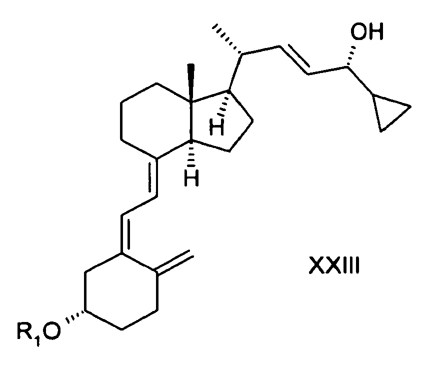

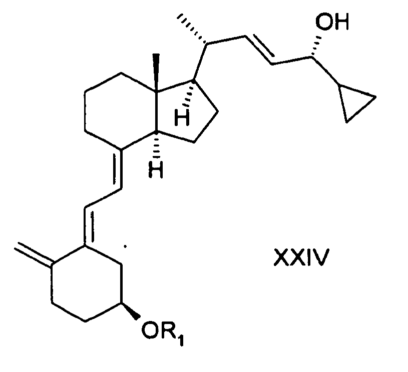

Compounds and intermediates of the present invention may comprise asymmetrically substituted (chiral) carbon atoms and carbon-carbon double bonds which may give rise to the existence of isomeric forms, e.g. enantiomers, diastereomers and geometric isomers. Epimers are known as diastereomers that have opposite configuration (R or S) at only one of multiple tetrahedral stereogenic centres in molecules having multiple stereogenic centres, such as the vitamin D analogues to which the present invention is directed. Designation of, for example, C-24 as the epimeric centre of a pair of enantiomers therefore implies that the configuration at the other stereogenic centres of the pair are identical. The process of converting one member of the epimeric pair to the other, by changing the stereochemistry at only one asymmetric center, can be referred to as epimerisation.

By the method of the present invention, a first epimer (either S or R), such as a 24 S or 24 R epimer of a vitamin-D analogue, alone or including some amount of a second epimer that has a configuration opposite to that of the first epimer, such as an epimer of a vitamin-D analogue that has a configuration opposite to that of the first epimer at C-24, is converted to a mixture that is relatively enriched in the second epimer, relative to the starting mixture. That is, the diastereomeric excess of the starting epimer or mixture of epimers is decreased from its initial value. The present invention provides a method for making mixed epimers, such as mixed epimers of a vitamin-D analogue, having a hydroxyl substituent on an asymmetric allylic carbon atom, such as a carbon atom at the C-24 position of a vitamin-D analogue, starting from an individual epimer or a mixture of epimers having an initial diastereomeric excess. The epimerisation process of the present invention may be continuous or preferably batchwise.

It is completely surprising that the novel epimerisation process achieves the desired epimerisation without significant formation of degradation products and thus without significant loss in yield. It is assumed that the epimerisation method of the present invention proceeds via protonation of the oxygen atom of the hydroxy group attached to the epimeric carbon atom. By nucleophilic attack of water to that carbon atom, either via a SN₁ or a SN₂ mechanism, the configuration at that carbon atom will be inverted. Under the reaction conditions of the present invention one might expect that the allylic alcohol containing side chain of the compounds to be epimerised would considerably degrade e.g by intermolecular ether formation or by rearrangement of intermediate carbocations formed upon the treatment with the acid. Furthermore, one would not expect vitamin-D analogues, especially those with an unprotected triene system, to tolerate the acidic conditions used in the process.

Any acid capable of protonating the oxygen atom of the hydroxy group attached to the epimeric carbon atom may be a suitable acid to achieve epimerisation. Such acids include phosphoric acid, organic acids, such as p-toluenesulfonic acid, and mineral acids, such as sulphuric acid, hydrobromic acid, hydroiodic acid, or hydrochloric acid. In a preferred embodiment of the present invention the acid is provided as a solution of a salt of an acid in water, such as a salt of a polyvalent mineral acid, such as a solution of sodium hydrogen sulphate in water. This has the advantage that the proper pH range can be obtained without tedious adjustment of the pH by titration and that dosing is facilitated under production conditions. Preferably the pH during contacting/epimerisation is below 3.0, such as below 2.0, e.g. 0 to 2.5, 1.1 to 1.9, such as 1.25, or 1.2 to 1.7, or 1.4 to 1.6.
Since the attack of water is involved in the epimerisation mechanism, water has to be present at least in traces, for example as residual water in organic solvents, or preferably as a co-solvent. A large molar excess of water may be preferable, such as an aqueous mixture of the substrate and an organic solvent. Preferably the epimerisation is carried out without nucleophiles present which may otherwise compete with the water. Preferred acids are thus acids with non-nucleophilic counter ions and preferred solvents are non-nucleophilic solvents.
Suitable solvents are any solvents compatible with the reaction conditions employed, or mixtures of such solvents, e.g. organic solvents. Non-limiting examples of solvents include hydrocarbons, such as toluene, ketones, such as acetone, esters, such as ethyl acetate, alcohols, such as methanol, ethanol, or 2-propanol, and ethers, such as *tert-*butyl methyl ether, tetrahydrofuran (THF), or dioxan. Solvents capable of dissolving the substrate, preferably water-miscible solvents and mixtures of such solvents are preferred. The preferred contacting of the compound to be epimerised is as a homogeneous mixture, such as a solution comprising the compound to be epimerised, an organic solvent or a mixture of organic solvents, water, and one or more acids. In a preferred embodiment of the present invention the epimerisation is carried out in a mixture of one or more water miscible solvents and water, preferably a mixture comprising water, acetone, and tetrahydrofuran, water and acetone or water and tetrahydrofuran.

In another embodiment of the invention the epimerisation reaction may be carried out under phase transfer conditions using a mixture of water and a water-immiscible solvent, such as toluene or xylene with a suitable phase transfer catalyst under acidic conditions.

In yet another embodiment of the present invention the epimerisation reaction may be carried out with an acidic ion-exchange resin as the acid, such as a strongly acidic cation resin.

In yet another embodiment of the present invention the epimerisation reaction may be carried out in a biphasic mixture of water and a water-immiscible solvent.

It was found that the reaction/contacting time and temperature may influence the formation of side products and hence the purity of the epimeric mixture obtained with the method described herein. Preferably, the contacting is carried out at a temperature of about 5-40°C, such as 10-35°C, such as 15-30°C, e.g. 20-25°C. The preferred contacting time is about 1-8 hours, such as 2-6 hours, e.g. 3-4 hours, such as 2-4 hours. Most preferably the contacting is carried out by contacting the epimeric mixture with an acid in the presence of water at 15-30°C for 2-4 hours.
The epimeric mixture of a compound to be epimerised in the sense of the present invention may comprise the epimers in any epimeric ratio (*R*) to (*S*) including the epimers in pure form. The present invention includes all possible epimeric ratios (*R*) to (*S*) even where not specifically indicated. The epimeric ratio will change during epimerisation until an equilibrium is reached. Since the epimeric centre usually will be in the vicinity of other chiral centres, said equilibrium may deviate from 50:50 due to diastereomeric interaction. However, the epimerisation may advantageously be stopped before reaching equilibrium at a point which provides the best compromise between reaction time, yield of the desired epimer, and the amount of impurities formed during epimerisation e.g. due to decomposition or degradation. In a presently preferred embodiment of the present invention, the epimerisation process is stopped when the epimeric mixture after epimerisation contains the C-24 epimers in an epimeric ratio (S):(R) of more than 29:71, such as more than 40:60. Since excessive contacting time during epimerisation may lead to losses of yield, the epimerisation may be advantageously stopped by increasing the pH of the reaction mixture by addition of a base, such as NaHCO₃, Na₂CO₃, NaOH, KOH, or K₂CO₃ to neutral or basic pH, such as to pH 7-10, e.g. pH 7.5-9.0, e.g. pH 8.0-8.5.
In a typical procedure, which is presently preferred, an epimeric mixture to be epimerised dissolved in an organic solvent or mixture thereof is concentrated and redissolved in a 1:1 (v:v) mixture of acetone and tetrahydrofuran. NaHSO₄ dissolved in water is added to the reaction mixture. After stirring of the mixture for about 2-4 hours at 20°C, the mixture is essentially neutralized with a base to stop the epimerisation.

The epimeric mixture after epimerisation may be separated into the pure epimers or into mixtures of said epimers enriched with one epimer, preferably by chromatography.
The separation, isolation, and purification methods of the present invention include, but are not limited to chromatography, such as adsorption chromatography (including column chromatography and simulated moving bed (SMB)), crystallisation, or distillation. The separation, isolation, and purification methods may be used subsequently and in combination.
Column chromatography, useful for the separation of vitamin D analogues or precursors for the synthesis of vitamin D analogues of the present invention is well known to those skilled in the art of pharmaceutical chemistry, organic chemistry or process chemistry. The technique employs a column packed with a stationary phase, for example silica, such as pretreated silica onto which sample to be separated is loaded. The sample is then eluted with a suitable eluent. Elution can be isocratic or so-called solvent programmed (gradient), wherein the composition of the eluent is varied regularly (e.g. linearly) or irregularly (e.g. stepwise over time. Pretreated silica gel, well known to a person skilled in the art of chromatography, is a suitable stationary phase. Elution with 5% (v:v) ethyl acetate in hexane or heptane followed by neat ethyl acetate is but one example of an elution program that produces the desired separation. Other suitable eluents will be deduced by the skilled person through routine methods of development, e.g. by using mixtures of heptane and ethylacetate of suitable polarity.
For chromatography, any combination of stationary phase (packing) and eluent that is capable of resolving the mixture of the epimers may be used. Such combinations can be readily determined by the skilled person by routine experimentation. An example of a preferred stationary phase is silica, such as treated silica.

The methods of the present invention are in particular useful for the recycling of the unwanted epimer in a production process of vitamin D derivatives, where such an unwanted epimer usually would have to be discarded. The present method includes multiple epimerisation and purification steps, i.e. the unwanted epimer, obtained by separation of a mixture after epimerisation, may be epimerised once more and then be separated for another epimerisation step, and so on. Various batches of epimers of different origin and chemical or diastereomeric purity may be pooled whenever appropriate prior to epimerisation.

### Synthetic Methods

The epimers of compounds comprising a side chain of general structure A or B of the present invention may be synthesized by methods well known to a person skilled in the art. General synthetic methods can for example be found in found in ["Vitamin D", D. Feldman, Ed., Academic Press, San Diego, USA, 1997] and [G.-D. Zhu et al., Chem. Rev. 1995, 95, 1877-1952] and references cited therein.

More specifically, the compounds of general structure IV can for example be synthesized via Diels-Alder reaction by treatment of a compound of general structure IIIa or IIIb with sulphur dioxide. The sulphur dioxide used can be liquid, gaseous or being dissolved in a suitable solvent. Suitable solvents for the Diels-Alder reaction are all solvents, which are compatible with the reaction conditions, such as alkanes, such as hexane or heptane, hydrocarbons, such as xylenes, toluene, ethers, such as diethyl ether or methyl-*tert*-butyl ether (MTBE), acetates, such as ethyl acetate or 2-propyl acetate, halogenated solvents such as dichloromethane, or mixtures of said solvents. In a preferred embodiment the solvent is toluene. In another preferred embodiment the solvent is a mixture of a water immiscible solvent and water, such as toluene and water. The reaction can also be carried out in neat sulphur dioxide without a solvent. A suitable reaction temperature of the process is -50°C to 60 °C, such as -30°C to 50°C, such as - 15°C to 40°C, such as -5°C to 30°C, such as 0°C to 35°C, such as 5°C to 30°C most such as 10°C to 25°C, such as 15°C to 20°C. Preferably the sulphur dioxide is used in excess (mol/mol), such as 5-100 molar excess, such as 7-30 molar excess, such as 10-15 molar excess. Any excess of unreacted sulphur dioxide can be removed from the reaction mixture by e.g. washing with aqueous base, such as aqueous sodium hydroxide or by distilling the sulphur dioxide off, optionally together with a solvent, optionally under reduced pressure. The compounds of general structure IV are usually obtained as a mixture of their epimers IVba and IVbb or IVaa and IVab.

Furthermore, compounds of general structure IV can be synthesized by enantioselective or diastereoselective reduction of their 24-keto-derivatives, such as with *N,N-*diethylaniline borane and optionally a chiral auxiliary, such as (1*S*,2*R*)-*cis*-1-amino-2-indanol. Said 24-keto-derivatives may be prepared via Diels-Alder reaction by treatment of a 24-keto derivative derived from structure III obtainable as outlined by M. J. Calverley, Tetrahedron, Vol. 43, No. 20, 1987 or in WO 87/00834.

Compounds of general structure I, IIa, IIb, IIIa or IIIb can for example be synthesised according to methods disclosed for example by M. J. Calverley, Tetrahedron, Vol. 43, No. 20, pp. 4609-4619, 1987 or in WO 87/00834.
For example compound IIIa, wherein both R₁ and R₂ are *tert*-butyldimethylsilyl which is described in these references may be deprotected with aqueous hydrofluoric acid in acetonitrile or tetrabutylammonium fluoride in THF to give a mixture of compounds wherein R₁ or R₂ are hydrogen, or to give a compound wherein R₁ and R₂ are hydrogen. This mixture of compounds can for example be separated by chromatography or crystallised as generally described herein. By reaction of said compounds of general structure IIIa, wherein R₁ and/or R₂ are hydrogen with a suitable protecting agent, new groups R₁ and/or R₂ can be introduced. Depending on the stoichiometry of the protecting agent used and the reaction conditions, mixtures of unprotected, monoprotected, and diprotected compounds can be obtained. Any intermediate of a mixture wherein one of R₁ or R₂ is hydrogen can then be isolated by chromatography and reacted with suitable protecting agent different from the first one used, to give compounds of general structure IIIa, wherein R₁ is different from R₂. In the same way R₁ and R₂ may be changed for other derivatives of the present invention, such as for compounds of general structures I, IIa, IIb, IIIb, IV, whenever appropriate.

The method for producing calcipotriol as described herein may be modified with regard to the order of the reaction steps, by omitting one or more reaction steps, or by introducing additional purification or reaction steps at any stage of the reaction sequence. The present invention includes all such modifications.

The method for producing calcipotriol as described herein includes further all variants, where the hydroxy protecting groups R₁ and/or R₂ for compounds or intermediates, where R₁ and/or R₂ are not hydrogen, are removed at any stage of the reaction sequence. Compounds or intermediates, wherein R₁ and/or R₂ are hydrogen may be protected with protecting agents at any stage of the reaction sequence, including protecting agents which yield other protecting groups than those removed earlier in the reaction sequence.

The present invention relates to all isomeric forms either in pure form or as mixtures thereof. The indication of a specific conformation or configuration either in the formulas or the names of compounds or intermediates of the present invention shall indicate that this specific conformation or configuration is a preferred embodiment of the invention. The indication of a specific conformation or configuration either in the formulas or the names of compounds or intermediates of the present invention shall include any other isomer than specifically indicated, either in pure form or as mixtures thereof, as another embodiment of the present invention.
The indication of an unspecific conformation or configuration either in the formulas or the names of compounds or intermediates of the present invention shall indicate that a mixture of these specific conformations or configurations is a preferred embodiment of the invention, but includes all specific conformations or configurations as embodiments although not explicitly stated. For example, the compound of general formula IVa preferably is a mixture of the epimers of general formula IVaa and IVab. The meaning of compound of general formula IVa includes the pure epimers IVaa and IVab as another embodiment of the invention.

The indication of an unspecific conformation or configuration either in the formulas or the names or numbering of compounds or intermediates of the present invention shall include any specific isomer although not specifically indicated in pure form, e.g. as another embodiment of the present invention.

Pure stereoisomeric forms of the compounds and the intermediates of this invention may be obtained by the application of procedures known in the art, such as by chromatography or crystallisation, or by stereoselective synthesis.

The retro Diels-Alder reaction of the mixture of compounds of general structure IV in the presence of a base to give compounds of general structure III may be carried out in all solvents, which are compatible with the reaction conditions, such as alkanes, such as hexane or heptane, hydrocarbons, such as xylenes, toluene, ethers, such as diethyl ether or methyl-*tert*-butyl ether (MTBE), acetates, such as ethyl acetate or 2-propyl acetate, halogenated solvents such as dichloromethane, water or mixtures of said solvents. Methods of said retro Diels Alder reaction are well known to a person skilled in the art of vitamin D synthesis (see e.g. M. J. Calverley, Tetrahedron, Vol. 43, No. 20, pp. 4609-4619, 1987 or in WO 87/00834). Preferred solvents are toluene, *tert*-butyl methyl ether, water, or mixtures thereof. Suitable bases to be used in the retro Diels-Alder reaction include, but are not limited to NaHCO₃, KHCO₃, Na₂CO₃, or K₂CO₃. In a preferred embodiment of the present invention, the base is aqueous NaHCO₃ and/or the retro Diels-Alder reaction is run above 70°C, such as between 70°C and 120 °C, most preferably between 74°C and 79°C.

Methods for the isomerisation of vitamin D derivatives and in particular compounds of general formula IIIa and/or IIIb to IIa and/or IIb are well known to a person skilled in the art of vitamin D synthesis. Reaction conditions can e.g. be found in M. J. Calverley, Tetrahedron, Vol. 43, No. 20, pp. 4609-4619, 1987 or in WO 87/00834 and references cited therein. In a preferred embodiment of the present invention, the isomerisation is a photo isomerisation, preferably with UV-light in the presence of a triplet sensitizer, e.g. anthracene, or even more preferably in the presence of 9-acetylanthracene.

Methods for the crystallization of e.g. vitamin D derivatives, and in particular calcipotriol or calcipotriol hydrate can for example be found in [M. J. Calverley, Tetrahedron, Vol. 43, No. 20, pp. 4609-4619, 1987, WO 94/15912; WO 2004/046097] and include crystallization from mixtures of ethylacetate and hexane of suitable polarity.

Compounds prepared by the epimerisation method of the present invention may be formulated together with pharmaceutically acceptable auxiliaries or excipients to give a pharmaceutical formulation or a medicament, such as a cream, an ointment or a gel.

### EXAMPLES

### General:

All chemicals, unless otherwise noted were from commercial sources. All melting points are uncorrected. For ¹H nuclear magnetic resonance (NMR) spectra (300 MHz) and ¹³C NMR (75.6 MHz) chemical shift values (δ) (in ppm) are quoted, unless otherwise specified; for deuteriochloroform solutions relative to internal tetramethylsilane (δ = 0.00) or chloroform (δ = 7.26) or deuteriochloroform (δ = 76.81 for ¹³C NMR) standard. The value of a multiplet, either defined (doublet (d), triplet (t), quartet (q)) or not (m) at the approximate mid point is given unless a range is quoted. Chromatography was performed on silica gel optionally using the flash technique. The TLC plates coated with silica gel were from Merck KGaA. Preferably the silica used for chromatography was from Merck KGaA Germany: LiChroprep® Si60 (15-25µm). Ethyl acetate, dichloromethane, or appropriate mixtures of ethyl acetate, dichloromethane, methanol, and petroleum ether (40-60) or heptane were used as eluents unless otherwise noted.

### Preparation 1:

### 1(S),3(R)-bis(tert-butyldimethylsilvloxy)-20(R)-(3'-cyclopropyl-3'-oxoprop-1'(E)-enyl)-9,10-secopregna-5(E),7(E),10(19)-triene SO₂-adducts

20(R),1(S),3(R)-bis(*tert*-butyldimethylsilyloxy)-20-(3'-cyclopropyl-3'-oxoprop-l'(*E*)-enyl)-9,10-secopregna-5(*E*),7(*E*),10(19)-triene (prepared according to the method described by M. J. Calverley, Tetrahedron, Vol. 43, No. 20, pp. 4609-4619, 1987) (20.0g) was dissolved in toluene (210 ml) at 20°C followed by the addition of water (40 ml) and SO₂ (20 ml) with stirring. When the reaction was judged to be complete by HPLC {Column LiChrosorb Si 60 5 µm 250x4mm from Merck, 2 ml/min flow, detection at 270nm & mass detection, hexane/ethyl acetate 9:1 (v:v)}, usually after 2-2.5 hours, a mixture of sodium hydroxide (27.7%, 60 ml) and water (80 ml) was added at 10-18°C until pH 6 of the reaction mixture. The toluene phase was separated and the solvent removed *in vacuo* without heating (preferably below 30°C) to give the two epimeric SO₂-adducts as a solid mixture. The two epimeric SO₂-adducts were separated by chromatography and a crystalline sample of the predominant form could be furthermore obtained by trituration of the solid mixture with methanol: ¹H NMR (CDCl₃) = 6.73 (dd,1H), 6.14 (d,1H), 4.69 (d,1H), 4.62 (d,1H), 4.35 (s,1H), 4.17 (m,1H), 3.92 (d,1H), 3.58 (d,1H), 2.61 (m,1H), 2.29 (m,1H), 2.2 - 1.2 (m,16H), 1.11 (d,3H), 1.05 (m,2H), 0.90 (m,2H), 0.87 (s,9H), 0.85 (s,9H), 0.68 (s,3H), 0.06 (s,3H), 0.05 (s,3H), 0.04 (s,3H), 0.02 (s,3H) ppm.

### Preparation 2:

### SO₂-adducts of 1(S),3(R)-bis(tert-butyl-dimethylsilyloxy)-20(R)-(3'-cyclopropyl-3'(S)-hydroxyprop-1'(E)-enyl)-9,10-secopregna-5(E),7(E),10(19)-triene

### (IVa: R₁, R₂ = tert-butyldimethylsilyl), and

### SO₂-adducts of 1(S),3(R)-bis(tert-butyl-dimethylsilvloxy)-20(R)-(3'-cyclopropyl-3'(R)-hdroxyprop-1'(E)-enyl)-9,10-secopregna-5(E),7(E),10(19)-triene

### (IVb: R₁, R₂ = tert-butyldimethylsilyl)

(*1S*,*2R*)-(-)-*cis*-1-amino-2-indanol (5.0g) was mixed with MTBE (160 ml) under a nitrogen atmosphere at 15-25°C followed by the addition of *N,N*-diethylaniline-borane (16.0 ml) at that temperature. The mixture was stirred until no more evolution of hydrogen could be observed. The mixture of SO₂-adducts of 1(S),3(R)-bis(*tert*-butyl-dimethylsilyloxy)-20(R)-(3'-cyclopropyl-3'-oxoprop-1'(*E*)-enyl)-9,10-secopregna-5(*E*),7(*E*),10(19)-triene obtained in preparation 1 was dissolved in toluene (160 ml) and MTBE (80 ml). This solution was added dropwise to said mixture at 15-25°C. The mixture was stirred for ca. 30 minutes after complete addition and then quenched with saturated aqueous NaHCO₃ at 10-15°C. The organic phase was separated and washed with 1 M hydrochloric acid (100 ml) at 0-10°C followed by washing with saturated aqueous NaHCO₃ (100 ml). The organic phase contained the SO₂-adducts of 1(S),3(R)-bis(*tert-*butyl-dimethylsilyloxy)-20(R)-(3'-cyclopropyl-3(S)'-hydroxyprop-1'(*E*)-enyl)-9,10-secopregna-5(*E*),7(*E*),10(19)-triene (IVa: R₁, R₂ = *tert*-butyldimethylsilyl), and the SO₂-adducts of 1(S),3(R)-bis(*tert*-butyl-dimethylsilyloxy)-20(R)-(3'-cyclopropyl-3'(R)-hydroxyprop-l'(*E*)-enyl)-9,10-secopregna-5(*E*),7(*E*),10(19)-triene (IVb: R₁, R₂ = *tert-*butyldimethylsilyl) in a molar ratio of 78:22 (IVa:IVb) as checked by HPLC-analysis of an aliquot after retro-Diels Alder reaction and analysis according to the method described in Example 2. Compound IVaa was isolated by chromatography on silica. ¹³C NMR (CDCl₃) IVa/ R₁, R₂ = *tert*-butyldimethylsilyl δ = 150.6, 137.6, 132.3, 129.3, 128.8, 109.0, 76.9, 67.3, 65.8, 64.5, 56.2, 56.1, 55.9, 46.0, 40.5, 40.0, 39.6, 34.1, 29.6, 27.4, 25.6, 25.5, 23.8, 21.8, 20.3, 17.8, 17.7, 17.4, 11.8, 2.8, 1.7, -4.7, -5.0, -5.0, -5.2 ppm.

### Preparation 3:

### 1(S),3(R)-bis(tert-butyl-dimethylsilyloxy)-20(R)-(3'-cyclopropyl-3(S)'-hydroxyprop-1'(E)-enyl)-9,10-secopregna-5(E),7(E),10(19)-triene

### (IIIa: R₁, R₂ = tert-butyldimethylsilyl), and

### 1(S),3(R)-bis(tert-butyl-dimethylsilyloxy)-20(R)-3'-cyclopropyl-3'(R)-hydroxyprop-1'(E)-enyl)-9,10-secopregna-5(E),7(E),10(19)-triene

### (IIIb: R₁, R₂ = tert-butyldimethylsilyl)

The organic phase from preparation 2 containing the SO₂-adducts of IVa (R₁, R₂ = *tert-*butyldimethylsilyl), and IVb (R₁, R₂ = *tert*-butyldimethylsilyl) was stirred vigorously with saturated aqueous NaHCO₃ (110 ml) and then heated (bath temperature ca. 90 °C) where the MTBE was distilled off. Conveniently the retro Diels-Alder reaction can be checked by HPLC {Column LiChrosorb Si 60 250x4mm from Merck, 1 ml/min flow, detection at 270nm, hexane/ethyl acetate 9:1.5 (v:v)}. After completion, usually 2-2.5 hours the reaction mixture was cooled to 15-25°C and the organic phase was separated, washed with saturated aqueous NaHCO₃ (110 ml) and water (100 ml). The solvent was removed *in vacuo* and the obtained oil (29 g) was dissolved in hexane (200 ml). The organic mixture was cooled to ca. -15°C, filtered over a short path of silica, and the remainder washed with hexane (ca. 100 ml). The hexane was removed *in vacuo* and the remaining oil, containing a mixture of 1(S),3(R)-bis(*tert*-butyl-dimethylsilyloxy)-20(R)-(3'-cyclopropyl-3(S)'-hydroxyprop-1'(*E*)-enyl)-9,10-secopregna-5(*E*),7(*E*),10(19)-triene (IIIa: R₁, R₂ = *tert*-butyldimethylsilyl), and 1(S),3(R)-bis(*tert*-butyl-dimethylsilyloxy)-20(R)-(3'-cyclopropyl-3'(R)-hydroxyprop-1'(*E*)-enyl)-9,10-secopregna-5(*E*),7(*E*),10(19)-triene (IIIb: R₁, R₂ = *tert*-butyldimethylsilyl) in a molar ratio of 78:22 (IIIa:IIIb) as checked by HPLC {Column LiChrosorb Si 60 5 µm 250x4mm from Merck, 1 ml/min flow, detection at 270nm, n-heptane/2-propanol 100:0.25 (v:v): RT IIIa ca. 14.3 min, IIIb: 11.9 min.}, which was purified by chromatography as described earlier by M. J. Calverley, Tetrahedron, Vol. 43, No. 20, pp. 4609-4619, 1987 or in WO 87/00834, to give 10.9 g (98.9 % HPLC purity) of IIIa R₁, R₂ = *tert*-butyldimethylsilyl after crystallisation from a mixture of hexane and methanol (and a small amount of triethylamine) in full accordance with the data described by M. J. Calverley in Tetrahedron, Vol. 43, No. 20, p. 4617, 1987 for compound 22. ¹³C NMR (CDCl₃) IIIa R₁, R₂ = *tert*-butyldimethylsilyl = 153.4, 142.9, 137.9, 135.2, 128.7, 121.5, 116.3, 106.4, 77.1, 70.0, 67.0, 56.2, 55.8, 45.7, 43.7, 40.2, 39.8, 36.3, 28.7, 27.5, 25.6, 25.6, 23.3, 22.0, 20.3, 18.0, 17.9, 17.4, 12.1, 2.9, 1.6, -5.0, -5.0, -5.1; IIIb/ R1, R2 = *tert-*butyldimethylsilyl = 153.5, 142.9, 137.6, 135.3, 128.7, 121.5, 116.3, 106.4, 76.8, 70.0, 67.0, 56.2, 56.0, 45.7, 43.8, 40.2, 39.7, 36.4, 28.7, 27.6, 25.7, 25.6, 23.3, 22.0, 20.3, 18.0, 17.9, 17.3, 12.1, 2.8, 1.6, -5.0, -5.1, -5.1 ppm.

### Preparation 4:

### 1(S),3(R)-bis(tert-butyl-dimethylsilyloxyl-20(R)-(3'-cyclopropyl-3(S)'-hydroxyprop-1'(E)-enyl)-9,10-secopregna-5(Z),7(E),10(19)-triene

### (IIa: R₁, R₂ = tert-butyldimethylsilyl)

1(S),3(R)-bis(*tert*-butyl-dimethylsilyloxy)-20(R)-(3'-cyclopropyl-3(S)'-hydroxyprop-1'(*E*)-enyl)-9,10-secopregna-5(*E*),7(*E*),10(19)-triene (IIIa: R₁, R₂ = *tert-*butyldimethylsilyl) obtained in preparation 3 was photosisomerised in toluene using a high pressure ultraviolet lamp at 20°C as described earlier by M. J. Calverley, Tetrahedron, Vol. 43, No. 20, pp. 4609-4619, 1987 or in WO 87/00834, except that 9-acetylanthracene was used instead of anthracene, to give 1(S),3(R)-bis(*tert*-butyl-dimethylsilyloxy)-20(R)-(3'-cyclopropyl-3(S)'-hydroxyprop-1'(*E*)-enyl)-9,10-secopregna-5(Z),7(E),10(19)-triene (IIa: R₁, R₂ = *tert*-butyldimethylsilyl) after chromatography in full accordance with the data described by M. J. Calverley in Tetrahedron, Vol. 43, No. 20, p. 4618, 1987 for compound 28.

### Example 1:

1(S),3(R)-bis(*tert*-butyl-dimethylsilyloxy)-20(R)-(3'-cyclopropyl-3'(R)-hydroxyprop-1'(*E*)-enyl)-9,10-secopregna-5(*E*),7(*E*),10(19)-triene (IIIb: R₁, R₂ = *tert-*butyldimethylsilyl) (10 mg), THF (1 ml) and aqueous sulphuric acid (0.4 ml, pH 1.7) were mixed in a test tube. After standing overnight at room temperature, HPLC-analysis (normal phase silica 20 cm analytical column, 4 ml/min., AcOEt/n-hexane 4/96 (v:v) indicates ca. 40 % of the starting epimer and ca. 45 % of 1(S),3(R)-bis(*tert*-butyl-dimethylsilyloxy)-20(R)-(3'-cyclopropyl-3(S)'-hydroxyprop-1'(*E*)-enyl)-9,10-secopregna-5(*E*),7(*E*),10(19)-triene (IIIa: R₁, R₂ = *tert*-butyldimethylsilyl) together with ca. 12% of two major and less polar impuirties of unkown structure.

### Example 2:

A mixture containing ca. 95% of 1(S),3(R)-bis(*tert*-butyl-dimethylsilyloxy)-20(R)-(3'-cyclopropyl-3'(R)-hydroxyprop-1'(*E*)-enyl)-9,10-secopregna-5(*E*),7(*E*),10(19)-triene (IIIb: R₁, R₂ = *tert*-butyldimethylsilyl) and <5 % of 1(S),3(R)-bis(*tert*-butyl-dimethylsilyloxy)-20(R)-(3'-cyclopropyl-3(S)'-hydroxyprop-1'(*E*)-enyl)-9,10-secopregna-5(*E*),7(*E*),10(19)-triene (IIIa: R₁, R₂ = *tert*-butyldimethylsilyl) (ca. 12 kg) was dissolved in a mixture of acetone (58 l) and THF (58.5 I). A solution of sodium hydrogen sulphate (0.3 kg) in water (20 l) (pH 1.58) was added with stirring keeping the temperature in the interval of 20-25°C. After 220 minutes a saturated solution of NaHCO₃ (6 l) was added to the reaction mixture. The organic solvents were mostly removed *in vacuo* and the remainder was redissolved in ethylacetate (100 l). The organic solution was then washed with brine (2 l saturated NaCl-solution in 180 l water). The ethylacetate was then removed *in vacuo*, the remainder was redissolved in hexane, and the epimeric mixture containing IIIa and IIIb (R₁, R₂ = *tert*-butyldimethylsilyl) in an epimeric ratio of 67:33 as checked by HPLC {Column LiChrosorb Si 60 5 µm 250x4mm from Merck, 1 ml/min flow, detection at 270nm, n-heptane/2-propanol 100:0.25 (v:v): RT IIIa ca. 14.3 min, IIIb: 11.9 min.} was separated by column chromatography on silica to give pure IIIa and IIIb (R₁, R₂ = *tert*-butyldimethylsilyl).

### Example 3:

1(S),3(R)-bis(*tert*-butyl-dimethylsilyloxy)-20(R)-(3'-cyclopropyl-3'(R)-hydroxyprop-1'(*E*)-enyl)-9,10-secopregna-5(*E*),7(*E*),10(19)-triene (IIIb: R₁, R₂ = *tert-*butyldimethylsilyl) (150 g), ethyl acetate (1.2 l), acetone (1.2 l) and aqueous sulphuric acid (121 ml, pH 1.25) were mixed with stirring. After stirring for ca. 35 min. at 27°C, HPLC-analysis indicates the epimeric mixture containing IIIa and IIIb (R₁, R₂ = *tert-*butyldimethylsilyl) in an epimeric ratio of 53:47.

### Example 4:

1(S),3(R)-bis(*tert*-butyl-dimethylsilyloxy)-20(R)-(3'-cyclopropyl-3'(R)-hydroxyprop-1'(*E*)-enyl)-9,10-secopregna-5(*E*),7(*E*),10(19)-triene (IIIb: R₁, R₂ = *tert-*butyldimethylsilyl) (10 g), tetrahydrofuran (10 ml), acetone (10ml) and phosphoric acid 1 ml, pH 1.48) were mixed with stirring. After stirring for ca. 1140 min. at 20°C, HPLC-analysis indicates the epimeric mixture containing IIIa and IIIb (R₁, R₂ = *tert-*butyldimethylsilyl) in an epimeric ratio of 39:61.

### Example 5:

1(S),3(R)-bis(*tert*-butyl-dimethylsilyloxy)-20(R)-(3'-cyclopropyl-3'(R)-hydroxyprop-1'(*E*)-enyl)-9,10-secopregna-5(*E*),7(*E*),10(19)-triene (IIIb: R₁, R₂ = *tert-*butyldimethylsilyl) (10 g), tetrahydrofuran (10 ml), acetone (10ml) and phosphoric acid 2 ml, pH 1.01) were mixed with stirring. After stirring for ca. 360 min. at 30°C, HPLC-analysis indicates the epimeric mixture containing IIIa and IIIb (R₁, R₂ = *tert-*butyldimethylsilyl) in an epimeric ratio of 38:62.

## Claims

1. A method of epimerising an epimer or epimeric mixture of a compound comprising a side chain of general structure A and/or B, 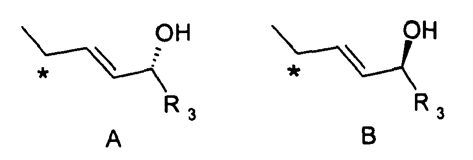 at the position of the carbon atom to which the hydroxy group and R₃ are attached to; by contacting said epimer or epimeric mixture with an acid in the presence of water;
wherein R₃ is (C₁-C₁₂)alkyl, (C₂-C₁₀)alkenyl, (C₂-C₂₀)alkynyl, or (C₃-C₂₀)cycloalkyl, optionally substituted with one or more substituents selected from the group consisting of (C₁-C₁₂)alkyl, -C(O)-O-(C₁-C₁₂)alkyl, halogen, hydroxy, methoxy, and ethoxy;
wherein the carbon marked with an asterisk is connected by a single bond to the C-17 carbon atom of a vitamin D analogue fragment;
or wherein the carbon marked with an asterisk is connected by a single bond to a fragment of a precursor for the synthesis of a vitamin D analogue at its C-17 analogous position;
wherein the fragment of a precursor for the synthesis of a vitamin D analogue is a fragment of a steroid ring system or a fragment of a CD ring system of a steroid;
wherein the fragment of the steroid ring system is represented by structure Q or R 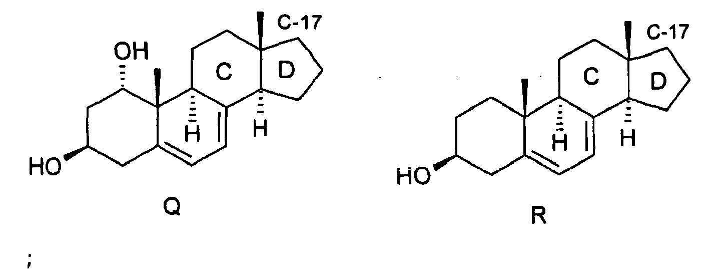 wherein the fragment of a CD ring system of a steroid is represented by structure E or P 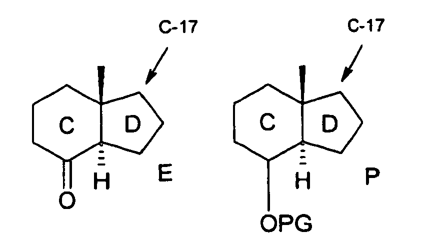 ; wherein PG represents hydrogen or a hydroxy protecting group;
wherein the vitamin D analogue fragment is represented by any one of the fragments F, G, H, J, K, or L 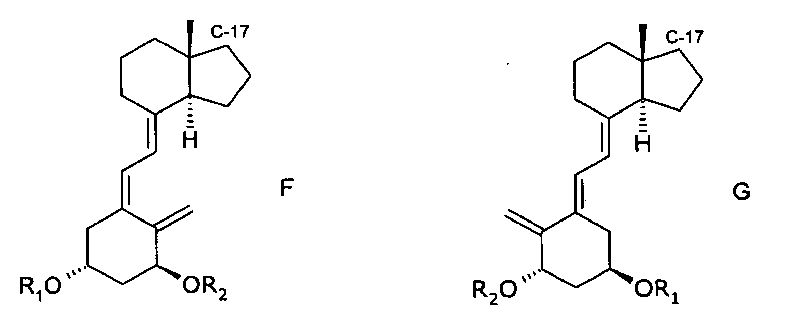 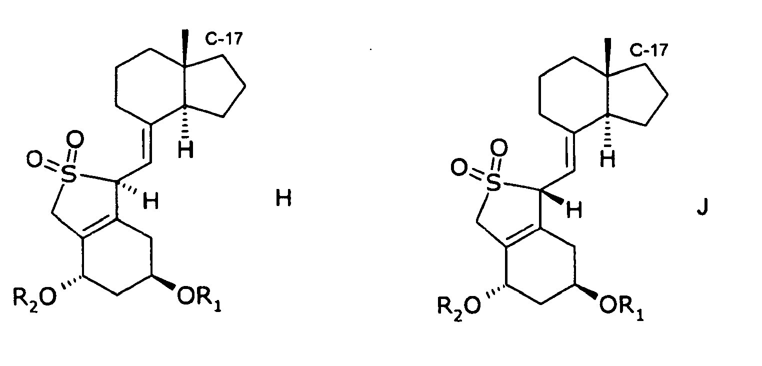 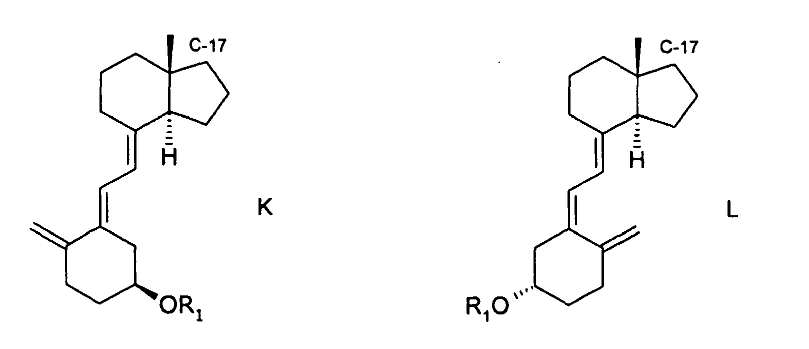 ; wherein R₁ and/or R₂ may be the same or different and represent hydrogen or a hydroxy protecting group.

2. The method according to claim 1, wherein R₃ is cyclopropyl.

3. The method according to claim 1, wherein R₃ is -C(CH₃)₂-C(O)-O-CH(CH₃)₂.

4. The method according to any one of claims 1-3, wherein the configuration at the carbon atom C-20 of the side chain marked with an asterisk is (R) and where the configuration at C-17 or the C-17 analogous position is the same as in natural vitamin D₃.

5. The method according to claim 1 of epimerising an epimer of a compound of structure IIIba or an epimeric mixture which comprises compounds IIIaa and IIIba, 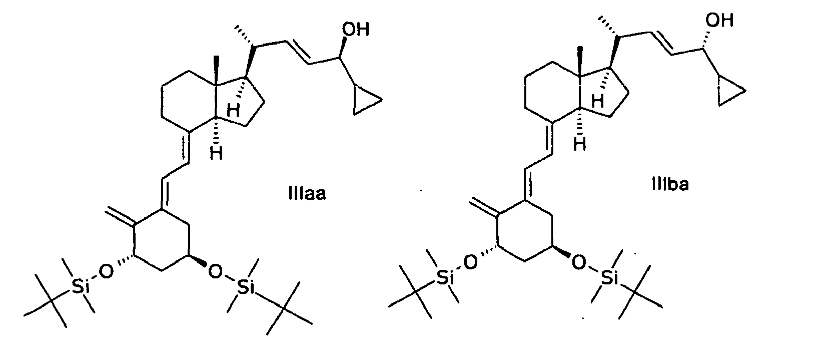 by contacting said epimer or epimeric mixture with an acid in the presence of water.

6. The method according to any one of claims 1-5 wherein the acid is a mineral acid.

7. The method according to claim 6 wherein the mineral acid is sulphuric acid.

8. The method according to any one of claims 1-7 wherein the acid is provided as a solution of a salt of a polyvalent acid in water.

9. The method according to claim 8 wherein the acid is provided as a solution of sodium hydrogen sulphate in water.

10. The method according to any one of claims 1-9 wherein the contacting is carried out at a pH below 3.0.

11. The method according to claim 10 wherein the contacting is carried out at a pH between 1.2 - 1.7.

12. The method according to any one of claims 1-11 wherein the contacting is carried out at a temperature of 15-30°C.

13. The method according to any one of claims 1-12 wherein the contacting is carried out for 2-4 hours.

14. The method according to any one of claims 1-13 wherein the contacting is carried out in a mixture of one or more water miscible solvents and water.

15. The method according to claim 14 wherein the contacting is carried out in a mixture comprising water, acetone, and tetrahydrofuran.

16. The method according to claims 1-15 wherein the epimeric mixture after epimerisation contains the C-24 epimers in an epimeric ratio (S):(R) of more than 29:71.

17. The method according to claim 16 wherein the epimeric mixture after epimerisation is separated by chromatography.

18. A method of preparing calcipotriol or calcipotriol monohydrate comprising, in one or more steps, the method of any one of claims 1-17.

19. A method for producing calcipotriol {(5Z, 7E, 22E, 24S)-24-cyclopropyl-9,10-secochola-5,7,10(19),22-tetraene-1α-3β-24-triol} or calcipotriol monohydrate comprising the steps of:
(i) epimerising a vitamin D-analogue of general structure IIb, 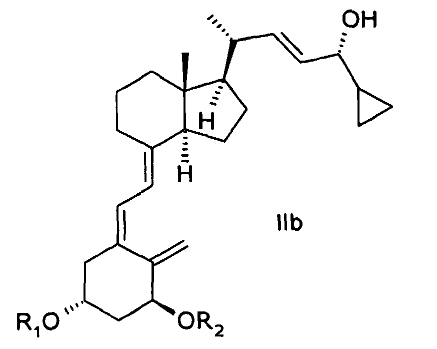 wherein R₁ and R₂ may be the same or different and represent hydrogen or a hydroxy protecting group,
with an acid in the presence of water to give a mixture of compounds of general structure IIa and IIb, wherein R₁ and R₂ are as defined above; 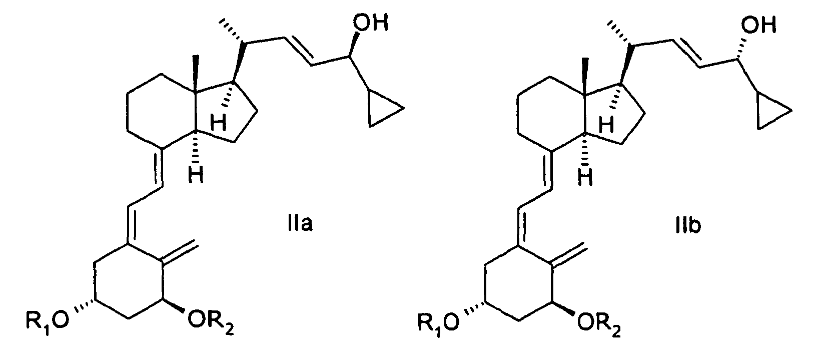
(ii) optionally separating the compound of general structure IIa from the mixture of compounds of general structure IIa and IIb, wherein R₁ and R₂ are as defined above;
(iii) when R₁ and/or R₂ are not hydrogen, removing the hydroxy protecting group(s) R₁ and/or R₂ of the compound of general structure IIa to generate calcipotriol ; and
(iv) optionally crystallising the calcipotriol from a mixture of an organic solvent and water to give calcipotriol monohydrate.

20. A method for producing calcipotriol {(5Z, 7E, 22E, 24S)-24-cyclopropyl-9,10-secochola-5,7,10(19),22-tetraene-1α-3β-24-triol} or calcipotriol monohydrate comprising the steps of:
(i) epimerising a vitamin D-analogue of general structure IIIb, 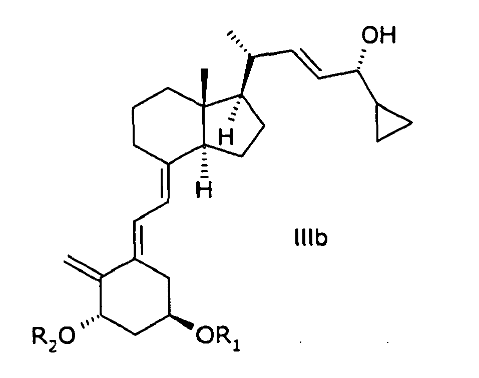 wherein R₁ and R₂ may be the same or different and represent hydrogen or a hydroxy protecting group,
with an acid in the presence of water to give a mixture of compounds of general structure IIIa and IIIb,
wherein R₁ and R₂ are as defined above; 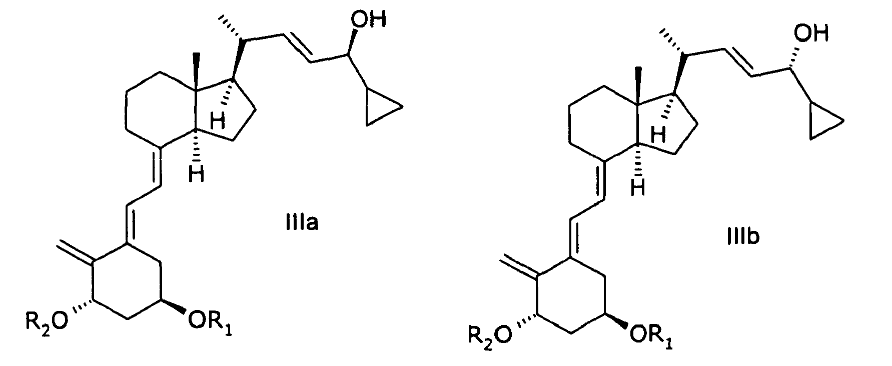
(ii) optionally separating the compound of general structure IIIa from the mixture of compounds of general structure IIIa and IIIb;
(iii) photoisomerising the compound of general structure IIIa to the compound of general structure IIa, 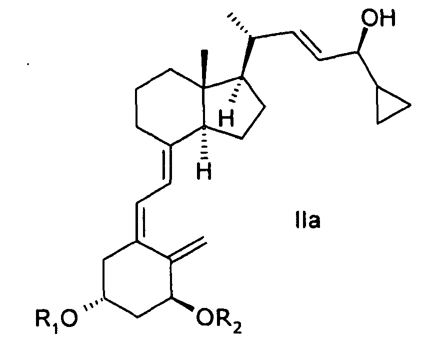 wherein R₁ and R₂ are as defined above;
(iv) when R₁ and/or R₂ are not hydrogen, removing the hydroxy protecting group(s) R₁ and/or R₂ of the compound of general structure IIa to generate calcipotriol; and
(v) optionally crystallising the calcipotriol from a mixture of an organic solvent and water to give calcipotriol monohydrate;
wherein steps (iii) and (iv) may be carried out in reverse order.

21. A method for producing calcipotriol {(5Z, 7E, 22E, 24S)-24-cyclopropyl-9,10-secochola-5,7,10(19),22-tetraene-1α-3β-24-triol} or calcipotriol monohydrate comprising the steps of:
(i) epimerising a vitamin D-analogue of general structure IVba and/or IVbb, 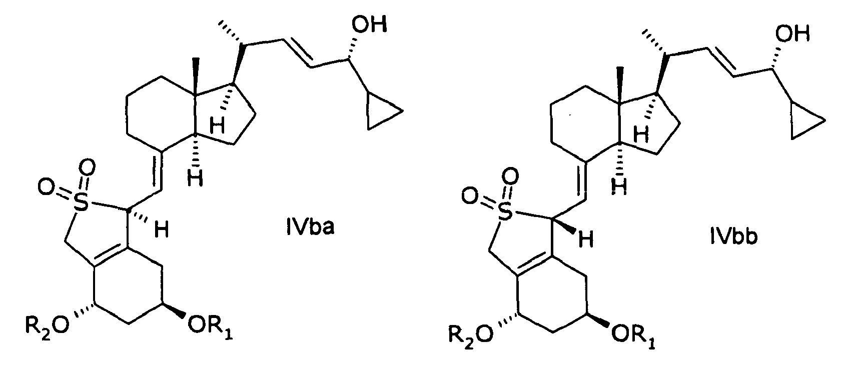 wherein R₁ and R₂ may be the same or different and represent hydrogen or a hydroxy protecting group,
with an acid in the presence of water to give a mixture of compounds of general structure IVba and/or IVbb and IVaa and/or IVab,
wherein R₁ and R₂ are as defined above; 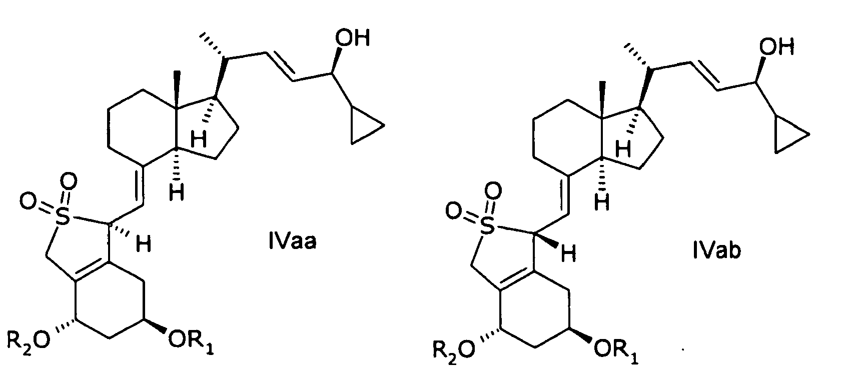
(ii) optionally separating the compounds of general structure IVaa and/or IVab from the reaction mixture;
(iii) heating the compounds of general structure IVaa and/or IVab above 60°C in the presence of a base to give a compound of general structure IIIa, 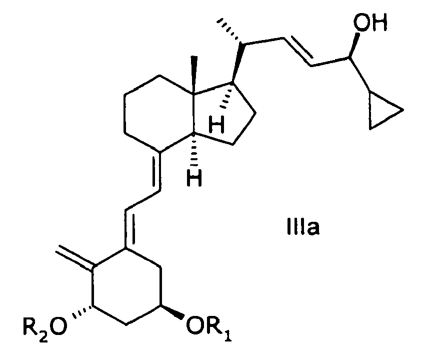 wherein R₁ and R₂ are as defined above;
(iv) photoisomerising the compound of general structure IIIa to the compound of general structure IIa, 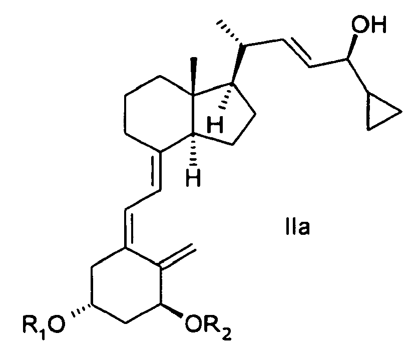 wherein R₁ and R₂ are as defined above;
(v) when R₁ and/or R₂ are not hydrogen, removing the hydroxy protecting group(s) R₁ and/or R₂ of the compound of general structure IIa to generate calcipotriol; and
(vi) optionally crystallising the calcipotriol from a mixture of an organic solvent and water to give calcipotriol monohydrate;
wherein steps (iv) and (v) may be carried out in reverse order.

22. A method according to claims 1, 19, 20 or 21, wherein R₁ and R₂ represent alkylsilyl or hydrogen.

23. A method according to claim 22, wherein R₁ and R₂ represent *tert*-butyldimethylsilyl.

24. The method according to claim 1 of epimerising an epimer of a compound of structure IIIba or an epimeric mixture which comprises compounds IIIaa and IIIba, 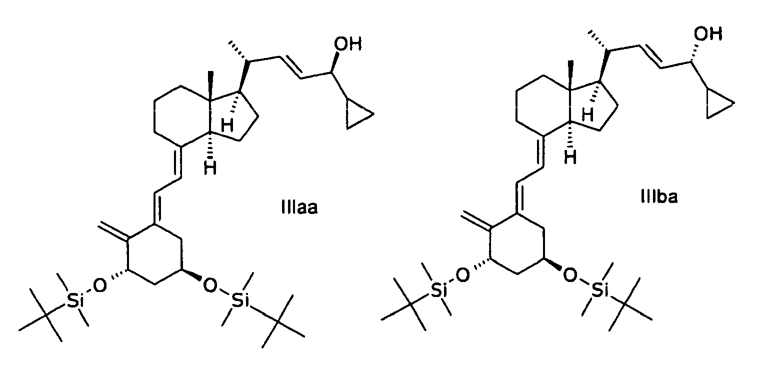 by contacting said epimer or epimeric mixture for 2-4 hours with an acid in the presence of water at a pH below 3.0 at a temperature of 15-30°C, wherein the acid is provided as a solution of sodium hydrogen sulphate in water.

25. Use of a method according to any one of claims 1-24 in the manufacture of calcipotriol or calcipotriol monohydrate.

26. A method for the manufacture of a pharmaceutical formulation or medicament containing calcipotriol or calcipotriol monohydrate, such as a cream, an ointment or a gel comprising the method according to any one of claims 1-24.

27. A method of preparing calcipotriol or calcipotriol monohydrate, the method comprising a method according to any one of claims 1-24.

## Patentansprüche

1. Verfahren zur Epimerisierung eines Epimers oder Epimergemisches einer eine Seitenkette der allgemeinen Struktur A und/oder B 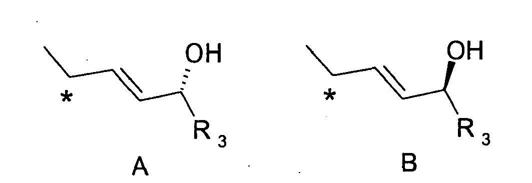 umfassenden Verbindung an der Position des Kohlenstoffatoms, an das die Hydroxygruppe und R₃ gebunden sind, wobei man eine Säure in Gegenwart von Wasser auf das Epimer oder Epimergemisch einwirken lässt,
wobei R₃ für (C₁-C₁₂)-Alkyl, (C₂-C₁₀)-Alkenyl, (C₂-C₂₀)-Alkinyl oder (C₃-C₂₀)-Cycloalkyl, gegebenenfalls mit einem oder mehreren, unter (C₁-C₁₂)-Alkyl, -C(O)-O-(C₁-C₁₂)-Alkyl, Halogen, Hydroxy, Methoxy und Ethoxy ausgewählten Substituenten substituiert, steht;
wobei der mit dem Stern **gekennzeichnete** Kohlenstoff über eine Einfachbindung mit dem C-17-Kohlenstoffatom eines Fragments eines Vitamin-D-Analogons verknüpft ist,
oder wobei der mit einem Stern **gekennzeichnete** Kohlenstoff über eine Einfachbindung mit einem Fragment einer Synthesevorstufe eines Vitamin-D-Analogons an dessen C-17-analogen Position verknüpft ist;
wobei das Fragment der Synthesevorstufe eines Vitamin-D-Analogons ein Fragment eines Steroidringsystems oder ein Fragment eines CD-Ringsystems eines Steroids ist;
wobei das Fragment des Steroidringsystems die Struktur Q oder R aufweist 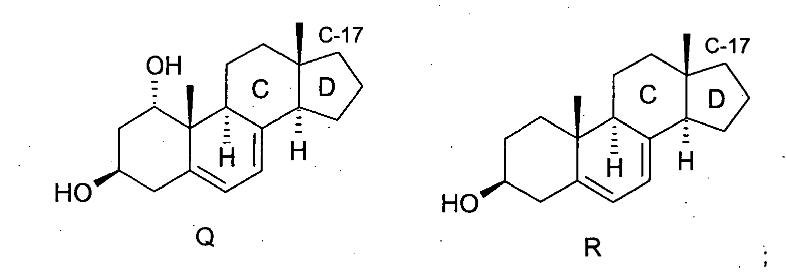 wobei das Fragment eines CD-Ringsystems eines Steroids die Struktur E oder P aufweist 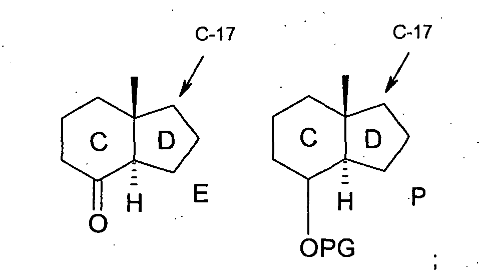 wobei PG für Wasserstoff oder eine Hydroxyschutzgruppe steht;
wobei das Fragment des Vitamin-D-Analogons eines von den Fragmenten F, G, H, J, K oder L ist 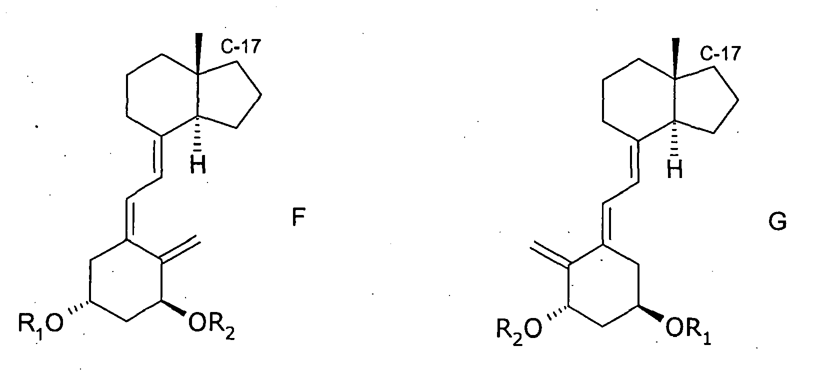 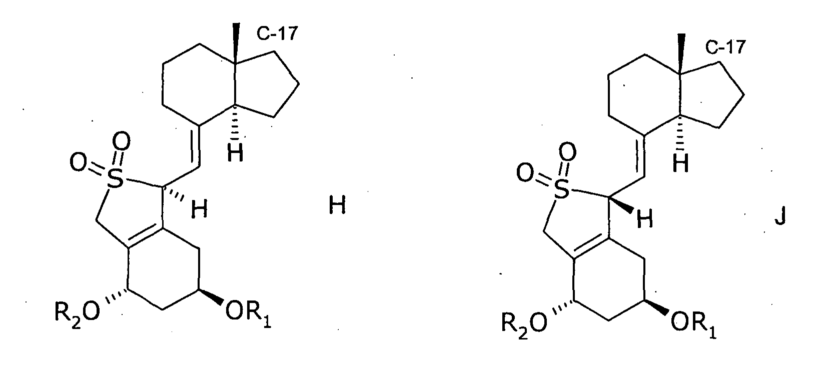 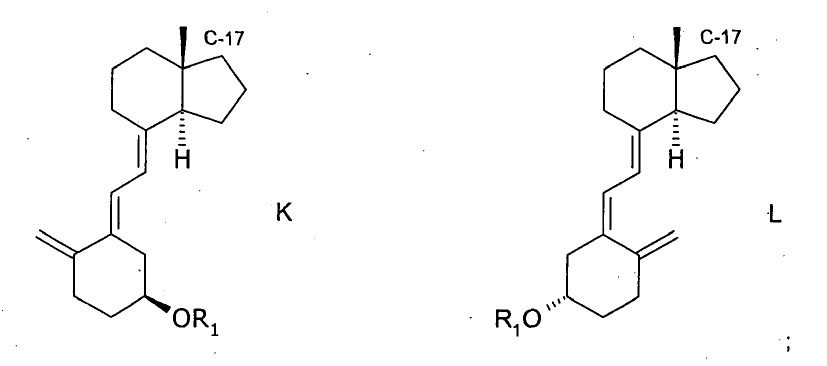 worin R₁ und/oder R₂ gleich oder verschieden sein können und für Wasserstoff oder eine Hydroxyschutzgruppe stehen.

2. Verfahren nach Anspruch 1, wobei R₃ Cyclopropyl ist.

3. Verfahren nach Anspruch 1, wobei R₃ -C(CH₃)₂-C(O)-O-CH(CH₃)₂ ist.

4. Verfahren nach einem der Ansprüche 1 bis 3, wobei das mit einem Stern **gekennzeichnete** Kohlenstoffatom C-20 der Seitenkette (R)-Konfiguration hat und wobei die Konfiguration am C-17 oder der C-17-analogen Position die gleiche ist wie im natürlichen Vitamin D₃.

5. Verfahren nach Anspruch 1 zur Epimerisierung eines Epimers einer Verbindung der Struktur IIIba oder eines Epimergemisches, welches Verbindungen IIIaa und IIIba 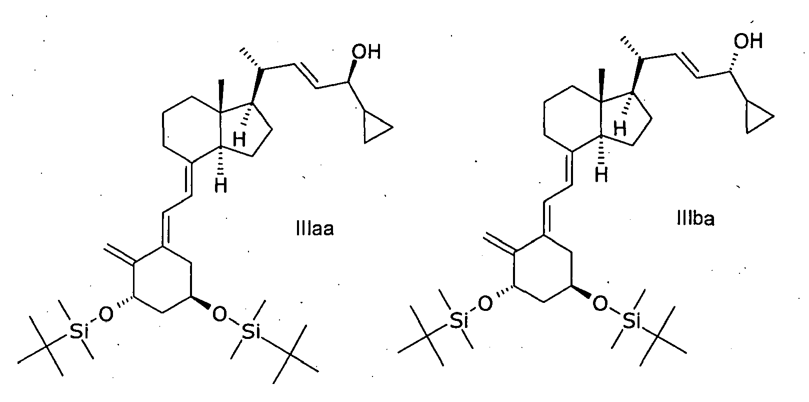 umfasst, wobei man eine Säure in Gegenwart von Wasser auf das Epimer oder Epimergemisch einwirken lässt.

6. Verfahren nach einem der Ansprüche 1 bis 5, wobei die Säure eine Mineralsäure ist.

7. Verfahren nach Anspruch 6, wobei die Mineralsäure Schwefelsäure ist.

8. Verfahren nach einem der Ansprüche 1 bis 7, wobei die Säure als Lösung eines Salzes einer polyvalenten Säure in Wasser eingesetzt wird.

9. Verfahren nach Anspruch 8, wobei die Säure als Lösung von Natriumhydrogensulfat in Wasser eingesetzt wird.

10. Verfahren nach einem der Ansprüche 1 bis 9, wobei das Einwirken bei einem pH unterhalb von 3,0 durchgeführt wird.

11. Verfahren nach Anspruch 10, wobei das Einwirken bei einem pH von 1,2 bis 1,7 durchgeführt wird.

12. Verfahren nach einem der Ansprüche 1 bis 11, wobei das Einwirken bei einer Temperatur von 15 bis 30 °C durchgeführt wird.

13. Verfahren nach einem der Ansprüche 1 bis 12, wobei das Einwirken für 2 bis 4 Stunden durchgeführt wird.

14. Verfahren nach einem der Ansprüche 1 bis 13, wobei das Einwirken in einem Gemisch von Wasser und einem oder mehreren mit Wasser mischbaren Lösungsmitteln durchgeführt wird.

15. Verfahren nach Anspruch 14, wobei das Einwirken in einem Wasser, Aceton und Tetrahydrofuran umfassenden Gemisch durchgeführt wird.

16. Verfahren nach Anspruch 1 bis 15, wobei das Epimergemisch nach Epimerisierung die C-24-Epimere in einem Epimerverhältnis (S):(R) von mehr als 29:71 enthält.

17. Verfahren nach Anspruch 16, wobei das Epimergemisch nach Epimerisierung chromatographisch aufgetrennt wird.

18. Verfahren zur Herstellung von Calcipotriol oder Calcipotriol-Monohydrat, das in einem oder mehreren Schritten das Verfahren nach einem der Ansprüche 1 bis 17 umfasst.

19. Verfahren zur Herstellung von Calcipotriol {(5Z,7E,22E,24S)-24-Cyclopropyl-9,10-secochola-5,7,10(19),22-tetraen-1α-3β-24-triol} oder Calcipotriol-Monohydrat, wobei das Verfahren die folgenden Schritte umfasst:
(i) Epimerisieren eines Vitamin-D-Analogons der allgemeinen Struktur IIb, 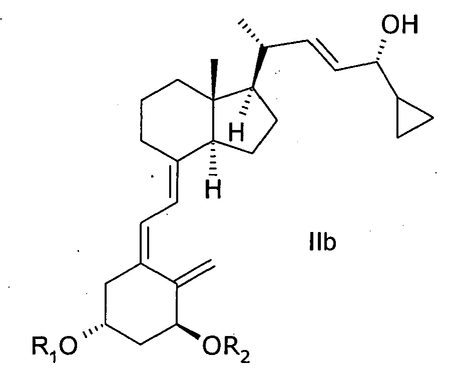 worin R₁ und R₂ gleich oder verschieden sein können und für Wasserstoff oder eine Hydroxyschutzgruppe stehen,
mit einer Säure in Gegenwart von Wasser, was ein Gemisch der Verbindungen der allgemeinen Struktur IIa und IIb, worin R₁ und R₂ wie oben definiert sind, ergibt; 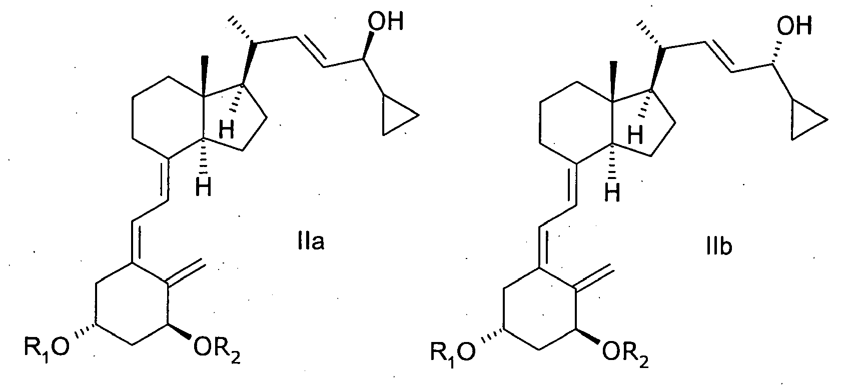
(ii) gegebenenfalls Abtrennen der Verbindung der allgemeinen Struktur IIa aus dem Gemisch der Verbindungen der allgemeinen Struktur IIa und IIb, worin R₁ und R₂ wie oben definiert sind;
(iii) falls R₁ und/oder R₂ nicht Wasserstoff sind, Abtrennen der Hydroxyschutzgruppe(n) R₁ und/oder R₂ der Verbindung der allgemeinen Struktur IIa, um Calcipotriol zu bilden; und
(iv) gegebenenfalls Kristallisieren des Calcipotriols aus einem Gemisch eines organischen Lösungsmittels und Wasser, was Calcipotriol-Monohydrat ergibt.

20. Verfahren zur Herstellung von Calcipotriol {(5Z,7E,22E,24S)-24-Cyclopropyl-9,10-secochola-5,7,10(19),22-tetraen-1α-3β-24-triol} oder Calcipotriol-Monohydrat, wobei das Verfahren die folgenden Schritte umfasst:
(i) Epimerisieren eines Vitamin-D-Analogons der allgemeinen Struktur IIIb, 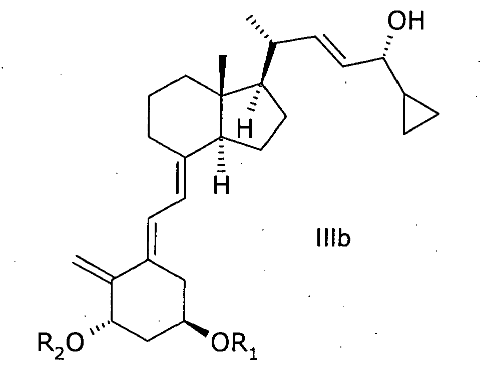 worin R₁ und R₂ gleich oder verschieden sein können und für Wasserstoff oder eine Hydroxyschutzgruppe stehen,
mit einer Säure in Gegenwart von Wasser, was ein Gemisch der Verbindungen der allgemeinen Struktur IIIa und IIIb,
worin R₁ und R₂ wie oben definiert sind, ergibt; 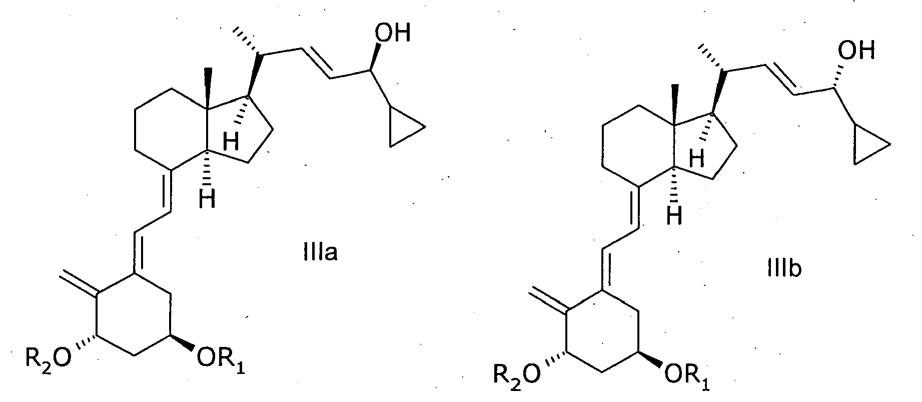
(ii) gegebenenfalls Abtrennen der Verbindung der allgemeinen Struktur IIIa aus dem Gemisch der Verbindungen der allgemeinen Struktur IIIa und IIIb;
(iii) Photoisomerisieren der Verbindung der allgemeinen Struktur IIIa zur Verbindung der allgemeinen Struktur IIa, 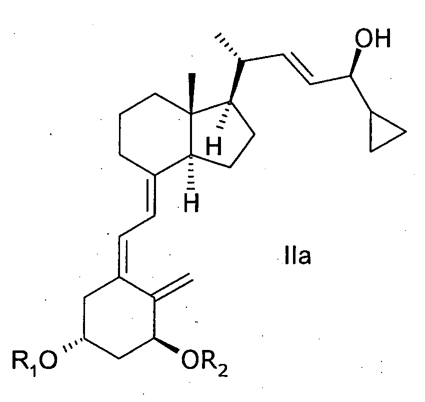 worin R₁ und R₂ wie oben definiert sind;
(iv) falls R₁ und/oder R₂ nicht Wasserstoff sind, Abtrennen der Hydroxyschutzgruppe(n) R₁ und/oder R₂ der Verbindung der allgemeinen Struktur IIa, um Calcipotriol zu bilden; und
(v) gegebenenfalls Kristallisieren des Calcipotriols aus einem Gemisch eines organischen Lösungsmittels und Wasser, was Calcipotriol-Monohydrat ergibt;
wobei die Schritte (iii) und (iv) in umgekehrter Reihenfolge durchgeführt werden können.

21. Verfahren zur Herstellung von Calcipotriol {(5Z,7E,22E,24S)-24-Cyclopropyl-9,10-secochola-5,7,10(19),22-tetraen-1α-3β-24-triol} oder Calcipotriol-Monohydrat, wobei das Verfahren die folgenden Schritte umfasst:
(i) Epimerisieren eines Vitamin-D-Analogons der allgemeinen Struktur IVba und/oder IVbb, 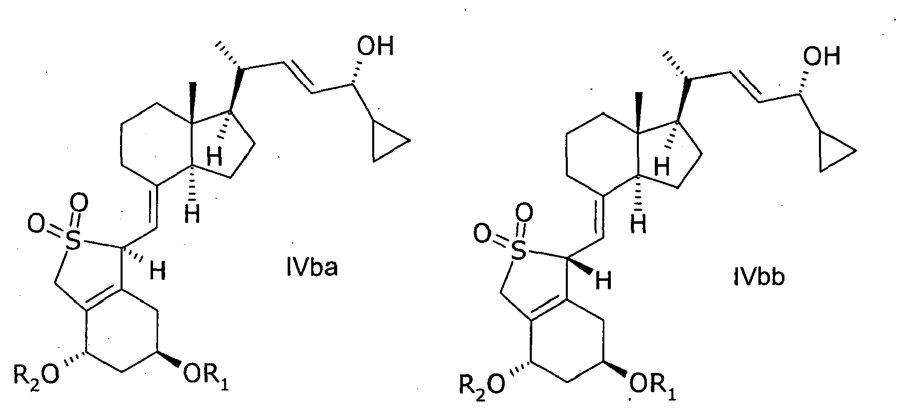 worin R₁ und R₂ gleich oder verschieden sein können und für Wasserstoff oder eine Hydroxyschutzgruppe stehen,
mit einer Säure in Gegenwart von Wasser, was ein Gemisch der Verbindungen der allgemeinen Struktur IVba und/oder IVbb und IVaa und/oder IVab, worin R₁ und R₂ wie oben definiert sind, ergibt; 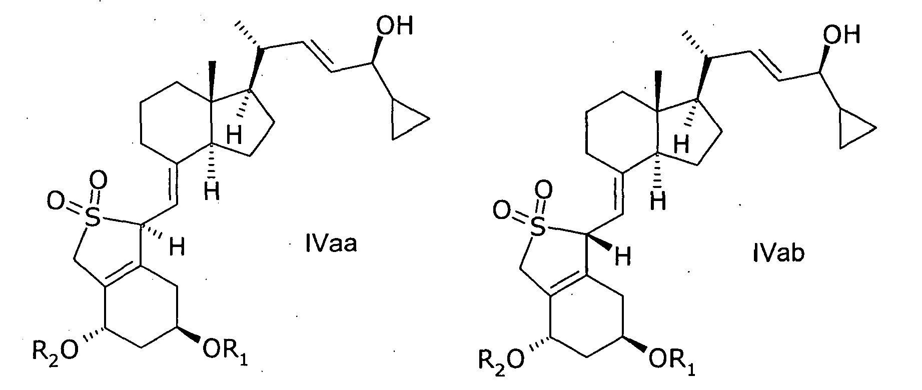
(ii) gegebenenfalls Abtrennen der Verbindungen der allgemeinen Formel IVaa und/oder IVab aus dem Reaktionsgemisch;
(iii) Erwärmen der Verbindungen der allgemeinen Struktur IVaa und/oder IVab oberhalb von 60 °C in Gegenwart einer Base, was eine Verbindung der allgemeinen Struktur IIIa, 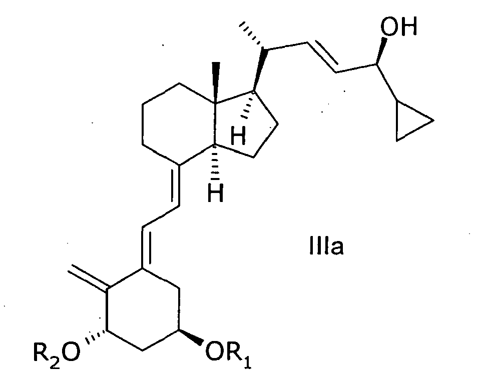 worin R₁ und R₂ wie oben definiert sind, ergibt;
(iv) Photoisomerisieren der Verbindung der allgemeinen Struktur IIIa zur Verbindung der allgemeinen Struktur IIa, 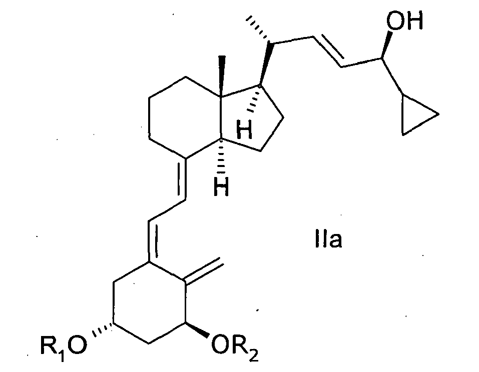 worin R₁ und R₂ wie oben definiert sind;
(v) falls R₁ und/oder R₂ nicht Wasserstoff sind, Abtrennen der Hydroxyschutzgruppe(n) R₁ und/oder R₂ der Verbindung der allgemeinen Struktur IIa, um Calcipotriol zu bilden; und
(vi) gegebenenfalls Kristallisieren des Calcipotriols aus einem Gemisch eines organischen Lösungsmittels und Wasser, was Calcipotriol-Monohydrat ergibt;
wobei die Schritte (iv) und (v) in umgekehrter Reihenfolge durchgeführt werden können.

22. Verfahren nach Anspruch 1, 19, 20 oder 21, wobei R₁ und R₂ für Alkylsilyl oder Wasserstoff stehen.

23. Verfahren nach Anspruch 22, wobei R₁ und R₂ *tert*-Butyldimethylsilyl sind.

24. Verfahren nach Anspruch 1 zur Epimerisierung eines Epimers einer Verbindung der Struktur IIIba oder eines die Verbindungen IIIaa und IIIba 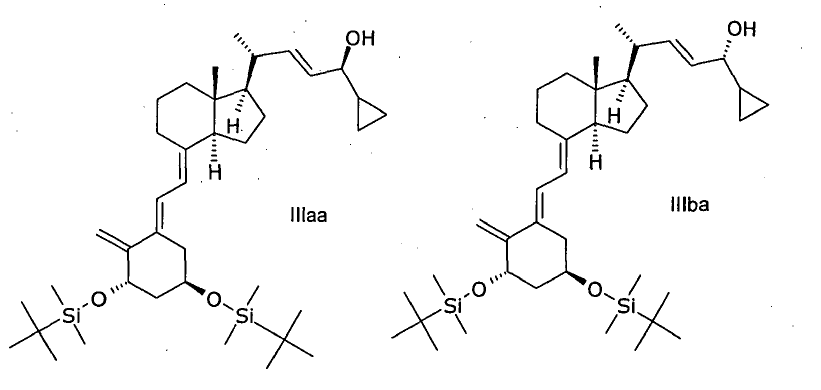 umfassenden Epimergemisches,
indem man eine Säure in Gegenwart von Wasser bei einem pH unterhalb von 3,0 bei einer Temperatur von 15 bis 30 °C auf das Epimer oder Epimergemisch 2 bis 4 Stunden einwirken lässt, wobei die Säure als Lösung von Natriumhydrogensulfat in Wasser eingesetzt wird.

25. Verwendung eines Verfahrens nach einem der Ansprüche 1 bis 24 bei der Herstellung von Calcipotriol oder Calcipotriol-Monohydrat.

26. Verwendung eines Verfahrens nach einem der Ansprüche 1 bis 24 bei der Herstellung einer pharmazeutischen Formulierung oder eines Medikaments, wie einer Creme, einer Salbe oder eines Gels, enthaltend Calcipotriol oder Calcipotriol-Monohydrat.

27. Calcipotriol oder Calcipotriol-Monohydrat, erhalten mit einem Prozess, der ein Verfahren nach einem der Ansprüche 1 bis 24 umfasst.

## Revendications

1. Procédé d'épimérisation d'un épimère ou d'un mélange épimère d'un composé comprenant une chaîne latérale de structure générale A et/ou B, 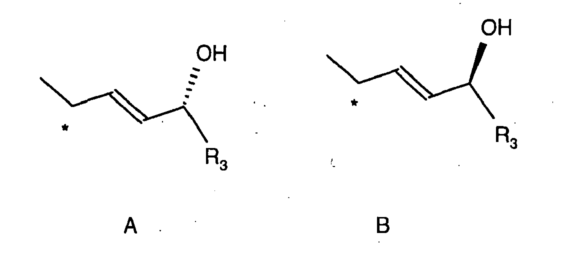 à la position de l'atome de carbone auquel le groupe hydroxy et R₃ sont fixés ;
par mise en contact dudit épimère ou mélange épimère avec un acide en présence d'eau ;
dans laquelle R₃ est un groupe alkyle en C₁-C₁₂, alcényle en C₂-C₁₀, alcynyle en C₂-C₂₀ ou cycloalkyle en C₃-C₂₀, facultativement substitué par un ou plusieurs substituants choisis parmi un groupe alkyle en C₁-C₁₂, -C(O)-O-(alkyle en C₁-C₁₂), un atome d'halogène, un groupe hydroxy, méthoxy et éthoxy ;
dans lequel le carbone marqué d'un astérisque est connecté par une liaison simple à l'atome de carbone C-17 d'un fragment d'analogue de vitamine D ;
ou dans lequel le carbone marqué d'un astérisque est connecté par une simple liaison à un fragment d'un précurseur pour la synthèse d'un analogue de vitamine D à sa position analogue C-17 ;
dans lequel le fragment d'un précurseur pour la synthèse d'un analogue de vitamine D est un fragment d'un système de cycle de stéroïde ou un fragment d'un système de cycle CD d'un stéroïde ;
dans lequel le fragment du système de cycle de stéroïde est représenté par la structure Q ou R 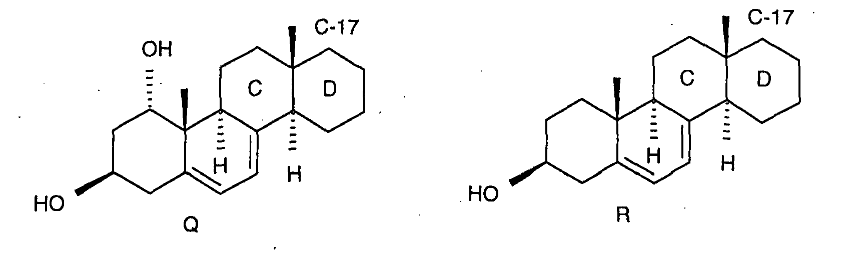 dans lequel le fragment d'un système de cycle CD d'un stéroïde est représenté par la structure E ou P 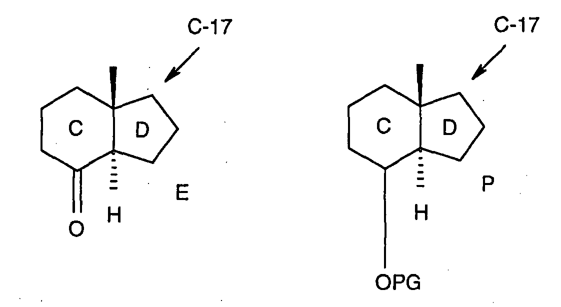 dans laquelle PG représente un atome d'hydrogène ou un groupe hydroxy-protecteur ;
dans lequel le fragment d'analogue de vitamine D est représenté par l'un quelconque des fragments F, G, H, J, K ou L 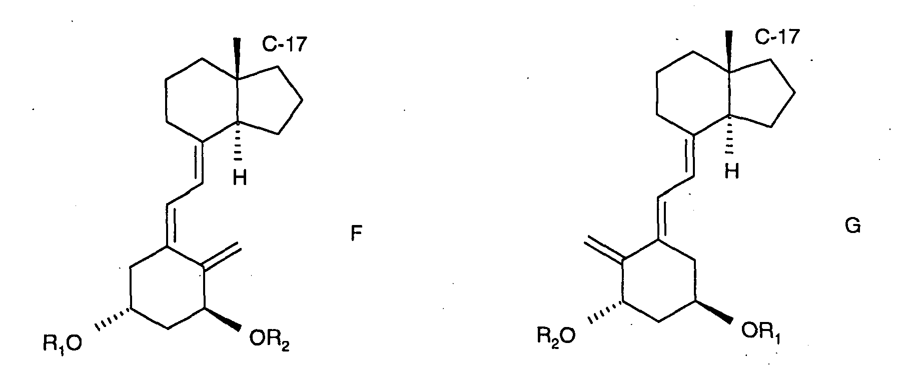 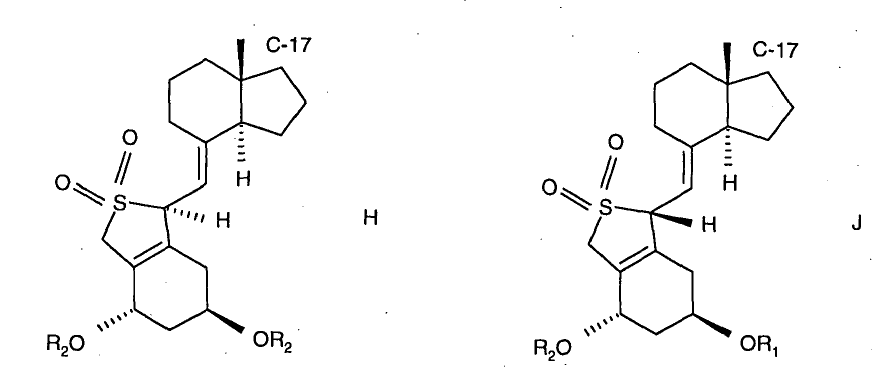 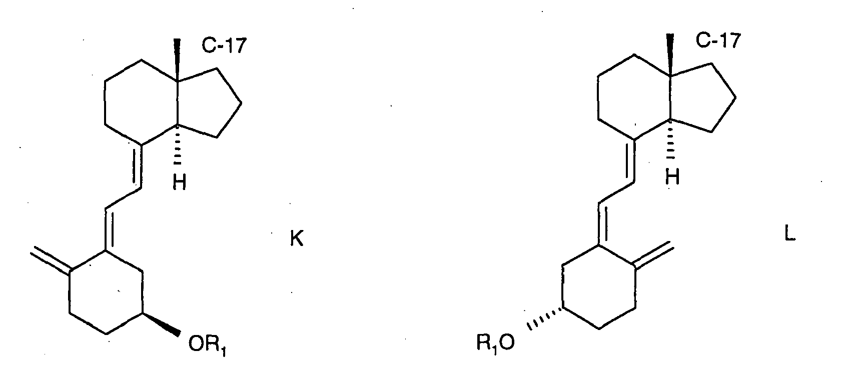 dans lesquels R₁ et/ou R₂ peuvent être identiques ou différents et représentent l'atome d'hydrogène ou un groupe hydroxy-protecteur.

2. Procédé selon la revendication 1, dans lequel R₃ est le groupe cyclopropyle.

3. Procédé selon la revendication 1, dans lequel R₃ est -C(CH₃)₂-C(O)-O-CH(CH₃)₂.

4. Procédé selon l'une quelconque des revendications 1-3, dans lequel la configuration à l'atome de carbone C-20 de la chaîne latérale marquée d'un astérisque est (*R*) et dans lequel la configuration en C-17 ou en la position analogue C-17 est identique à celle dans la vitamine D₃ naturelle.

5. Procédé selon la revendication 1 d'épimérisation d'un épimère d'un composé de la structure IIIba ou d'un mélange épimère qui comprend les composés IIIaa et IIIba, 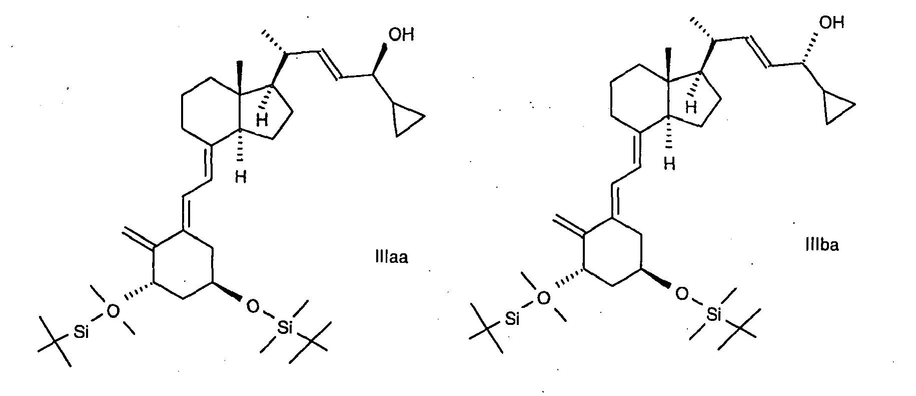 par mise en contact dudit épimère ou mélange épimère avec un acide en présence d'eau.

6. Procédé selon l'une quelconque des revendications 1-5, dans lequel l'acide est un acide minéral.

7. Procédé selon la revendication 6, dans lequel l'acide minéral est l'acide sulfurique.

8. Procédé selon l'une quelconque des revendications 1-7, dans lequel l'acide est fourni comme une solution d'un sel d'un acide polyvalent dans l'eau.

9. Procédé selon la revendication 8, dans lequel l'acide est fourni comme une solution d'hydrogénosulfate de sodium dans l'eau.

10. Procédé selon l'une quelconque des revendications 1-9, dans lequel la mise en contact est réalisée à un pH inférieur à 3,0.

11. Procédé selon la revendication 10, dans lequel la mise en contact est réalisée à un pH compris entre 1,2 et 1,7.

12. Procédé selon l'une quelconque des revendications 1-11, dans lequel la mise en contact est réalisée à une température de 15-30°C.

13. Procédé selon l'une quelconque des revendications 1-12, dans lequel la mise en contact est réalisée pendant 2-4 h.

14. Procédé selon l'une quelconque des revendications 1-13, dans lequel la mise en contact est réalisée dans un mélange d'un ou plusieurs solvants miscibles dans l'eau et d'eau.

15. Procédé selon la revendication 14, dans lequel la mise en contact est réalisée dans un mélange comprenant de l'eau, de l'acétone et du tétrahydrofurane.

16. Procédé selon les revendications 1-15, dans lequel le mélange épimère après l'épimérisation contient les épimères C-24 dans un rapport épimère (S):(R) supérieur à 29 : 71.

17. Procédé selon la revendication 16, dans lequel le mélange épimère est après l'épimérisation séparé par chromatographie.

18. Procédé de préparation de calcipotriol ou de monohydrate de calcipotriol comprenant, en une ou plusieurs étapes, le procédé selon l'une quelconque des revendications 1-17.

19. Procédé pour la production de calcipotriol {(5Z, 7E, 22E, 24S)-24-cyclopropyl-9,10-secochola-5,7,10(19),22-tétraène-1α-3β-24-triol} ou de monohydrate de calcipotriol comprenant les étapes :
(i) d'épimérisation d'un analogue de vitamine D de structure générale IIb, 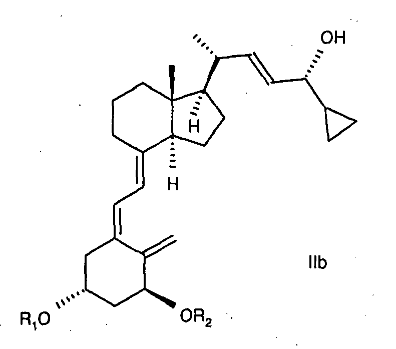 dans laquelle R₁ et R₂ peuvent être identiques ou différents et représentent un atome d'hydrogène ou un groupe hydroxy-protecteur, avec un acide en présence d'eau pour fournir un mélange de composés de structure générale IIa et IIb, dans laquelle R₁ et R₂ sont comme définis ci-dessus ; 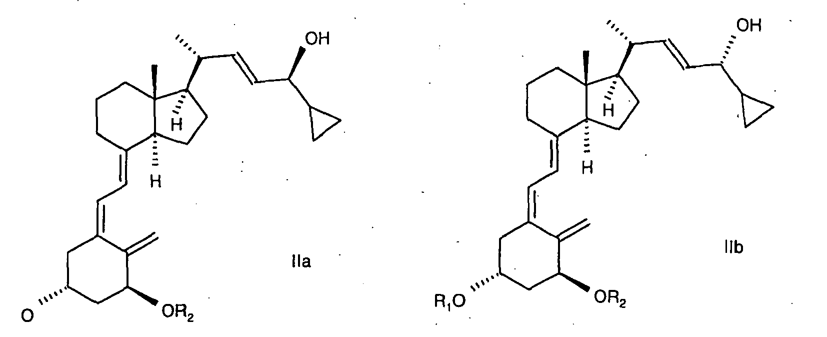
(ii) de séparation facultative du composé de structure générale IIa du mélange de composés de structure générale IIa et IIb, dans laquelle R₁ et R₂ sont comme définis ci-dessus ;
(iii) lorsque R₁ et/ou R₂ ne sont pas l'atome d'hydrogène, d'élimination du (des) groupe(s) hydroxy-protecteur(s) R₁ et/ou R₂ du composé de structure générale IIa pour produire du calcipotriol ; et
(iv) de cristallisation facultative du calcipotriol à partir d'un mélange d'un solvant organique et d'eau pour fournir du monohydrate de calcipotriol.

20. Procédé de production de calcipotriol {(5Z, 7E, 22E, 24S)-24-cyclopropyl-9,10-secochola-5,7,10(19),22-tétraène-1α-3β-24-triol} ou de monohydrate de calcipotriol comprenant les étapes :
(i) d'épimérisation d'un analogue de vitamine D de structure générale IIIb, 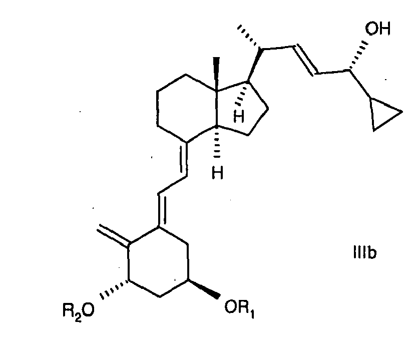 dans laquelle R₁ et R₂ peuvent être identiques ou différents et représentent un atome d'hydrogène ou un groupe hydroxy-protecteur, avec un acide en présence d'eau pour fournir un mélange de composés de structure générale IIIa et IIIb,
dans laquelle R₁ et R₂ sont comme définis ci-dessus ; 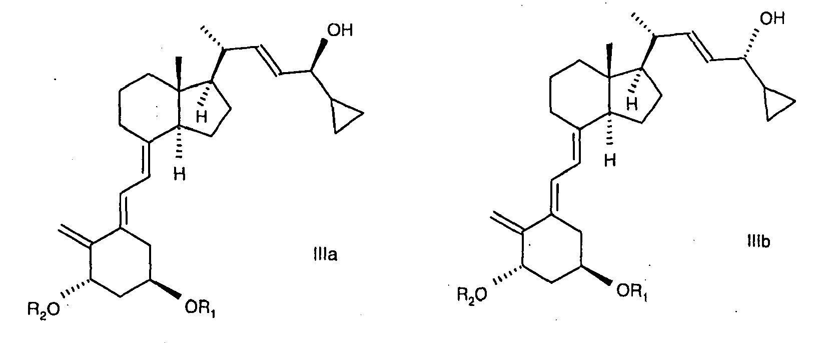
(ii) de séparation facultative du composé de structure générale IIIa du mélange de composés de structure générale IIIa et IIIb ;
(iii) de photoisomérisation du composé de structure générale IIIa en le composé de structure générale IIa, 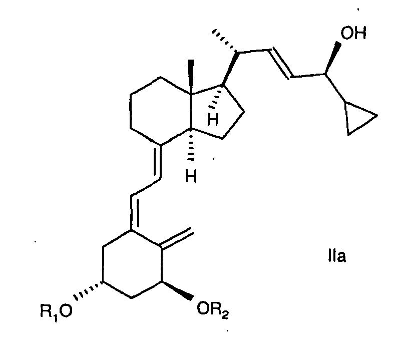 dans laquelle R₁ et R₂ sont comme définis ci-dessus ;
(iv) lorsque R₁ et/ou R₂ ne sont pas l'atome d'hydrogène, d'élimination du(des) groupe(s) hydroxy protecteur(s) R₁ et/ou R₂ du composé de structure générale IIa pour produire du calcipotriol ; et
(v) de cristallisation facultative du calcipotriol à partir d'un mélange d'un solvant organique et d'eau pour fournir du monohydrate de calcipotriol ;
dans lequel les étapes (iii) et (iv) peuvent être réalisées dans l'ordre inverse.

21. Procédé pour la production de calcipotriol {(5Z, 7E, 22E, 24S)-24-cyclopropyl-9,10-secochola-5,7,10(19),22-tétraène-1α-3β-24-triol} ou de monohydrate de calcipotriol comprenant les étapes :
(i) d'épimérisation d'un analogue de vitamine D de structure générale IVba et/ou IVbb 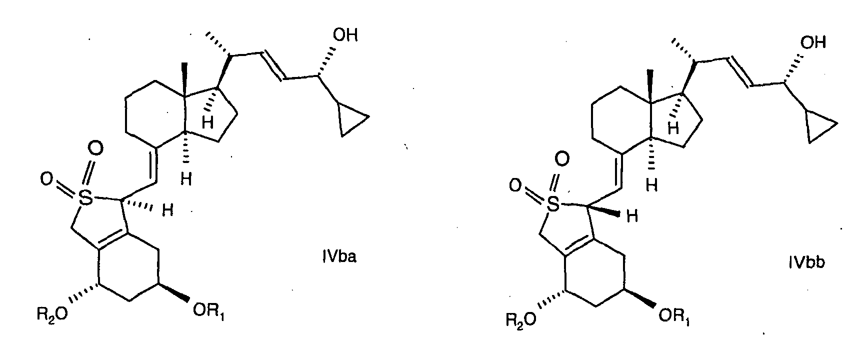 dans laquelle R₁ et R₂ peuvent être identiques ou différents et représentent un atome d'hydrogène ou un groupe hydroxy-protecteur, avec un acide en présence d'eau pour fournir un mélange de composés de structure générale IVba et/ou IVbb et IVaa et/ou IVab, dans laquelle R₁ et R₂ sont comme définis ci-dessus ; 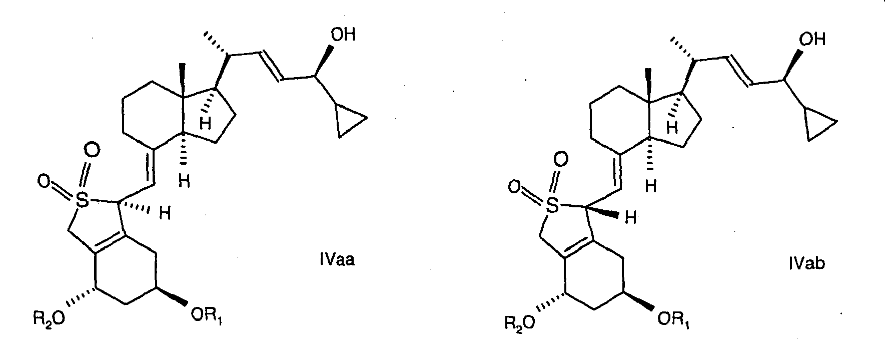
(ii) de séparation facultative des composés de structure générale IVaa et/ou IVab du mélange réactionnel ;
(iii) de chauffage des composés de structure générale IVaa et/ou IVab au-dessus de 60°C en présence d'une base pour fournir un composé de structure générale IIIa, 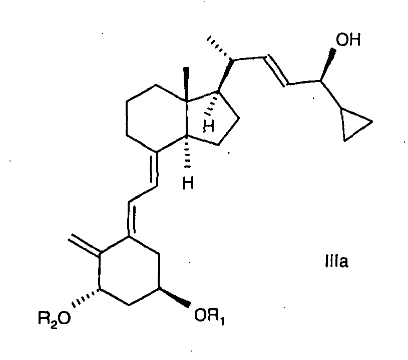 dans laquelle R₁ et R₂ sont comme définis ci-dessus ;
(iv) de photoisomérisation du composé de structure générale IIIa en le composé de structure générale IIa, 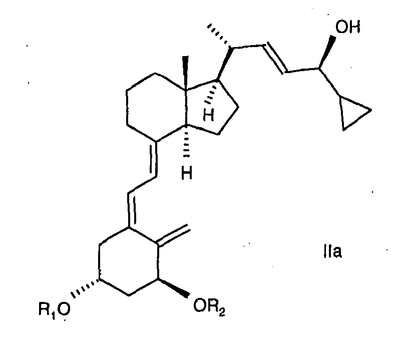 dans laquelle R₁ et R₂ sont comme définis ci-dessus ;
(v) lorsque R₁ et/ou R₂ ne sont pas l'atome d'hydrogène, d'élimination du(des) groupe(s) hydroxy protecteur(s) R₁ et/ou R₂ du composé de structure générale IIa pour produire du calcipotriol ; et
(vi) de cristallisation facultative du calcipotriol à partir d'un mélange d'un solvant organique et d'eau pour fournir du monohydrate de calcipotriol ;
dans lequel les étapes (iv) et (v) peuvent être réalisées dans l'ordre inverse.

22. Procédé selon les revendications 1, 19, 20 ou 21, dans lequel R₁ et R₂ représentent un groupe alkylsilyle ou un atome d'hydrogène.

23. Procédé selon la revendication 22, dans lequel R₁ et R₂ représentent le groupe *tert*-butyldiméthylsilyle.

24. Procédé selon la revendication 1 d'épimérisation d'un épimère d'un composé de structure IIIba ou d'un mélange épimère qui comprend les composés IIIaa et IIIba, 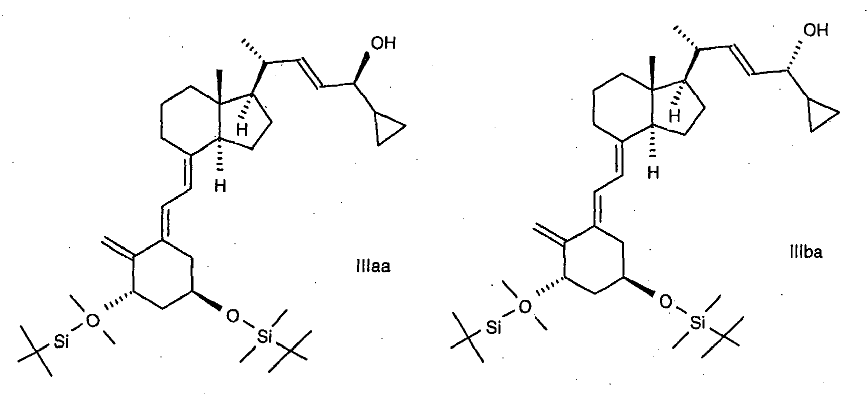 par mise en contact dudit épimère ou mélange épimère pendant 2-4 h avec un acide en présence d'eau à un pH inférieur à 3,0 à une température de 15-30°C, dans lequel l'acide est fourni comme une solution d'hydrogénosulfate de sodium dans l'eau.

25. Utilisation d'un procédé selon l'une quelconque des revendications 1-24 dans la fabrication de calcipotriol ou de monohydrate de calcipotriol.

26. Procédé pour la fabrication d'une formulation pharmaceutique ou d'un médicament contenant du calcipotriol ou du monohydrate de calcipotriol, tel qu'une crème, un onguent ou un gel comprenant le procédé selon l'une quelconque des revendications 1-24.

27. Procédé de préparation de calcipotriol ou de monohydrate de calcipotriol, le procédé comprenant un procédé selon l'une quelconque des revendications 1-24.
